# EUROPÄISCHE PATENTSCHRIFT

(11) **EP 0 065 732 B1**
(45) Veröffentlichungstag und Bekanntmachung des Hinweises auf die Patenterteilung: **21.08.1985**
(21) Anmeldenummer: 82104301.5
(22) Anmeldetag: 17.05.1982
(51) Int. Cl.: C09B 62/09, C09B 62/513, D06P 3/66, D06P 3/10

(54) **Wasserlösliche Disazoverbindungen, Verfahren zu deren Herstellung und ihre Verwendung als Farbstoffe**
Water soluble disazo compounds, process for their preparation and their use as dyestuffs
Composés disazoiques, solubles dans l'eau procédé pour leur préparation et leur utilisation comme colorants

(30) Priorität: 21.05.1981 DE 3120187
(43) Veröffentlichungstag der Anmeldung: 01.12.1982
(73) Patentinhaber: HOECHST AKTIENGESELLSCHAFT, 65926 Frankfurt am Main (DE)
(72) Erfinder: Meininger, Fritz, Dr., D-6230 Frankfurt am Main 80 (DE); Hoyer, Ernst, Dr., D-6230 Frankfurt am Main 80 (DE); Fass, Rudolf, D-6233 Kelkheim (Taunus) (DE)

## Beschreibung

Die vorliegende Erfindung betrifft neue wasserlösliche Disazoverbindungen, die die allgemeine Formel (1) 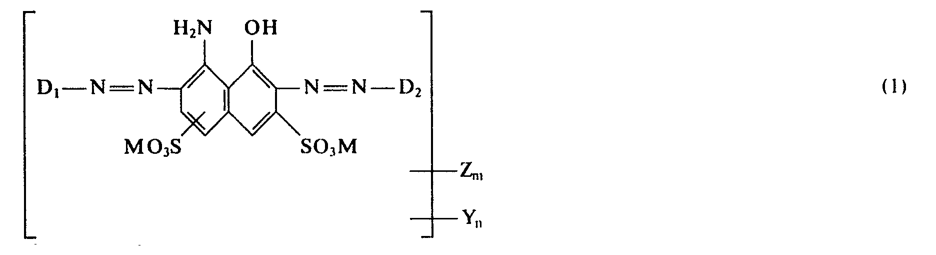 besitzen; in dieser bedeuten:
Dᵢ und D₂ stellt jedes den Phenylrest oder einen Naphthylrest dar, die beide durch eine Gruppe des nachstehend definierten Formelrestes Y oder Formelrestes Z substituiert sind und zusätzlich durch einen oder zwei Substituenten aus der Gruppe Sulfo, Chlor, Brom, niederes Alkyl, wie Äthyl und insbesondere Methyl, und niederes Alkoxy, wie Äthoxy und insbesondere Methoxy, substituiert sein können,

wobei D₁ und D₂ zueinander gleiche oder voneinander verschiedene Bedeutungen besitzen können;

Z ist eine Gruppe der Formel (2a), (2b), (2c), (2d), (2e) oder (2f) 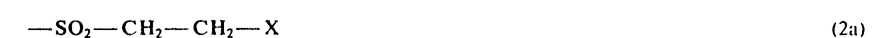 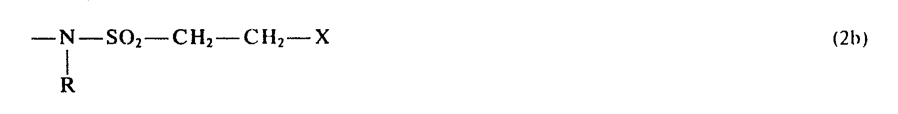 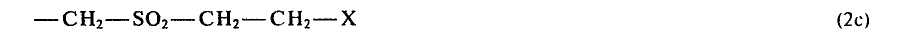 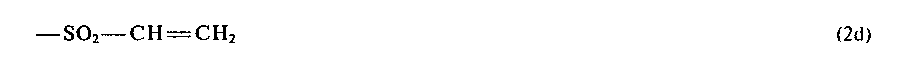 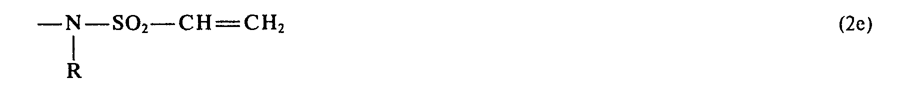 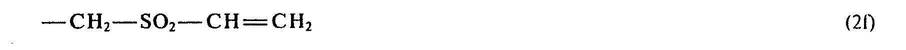 in welchen
R eine Alkylgruppe von 1 bis4 C-Atomen, vorzugsweise die Methylgruppe, und
X ein Chloratom oder die Acetyloxygruppe oder die Thiosulfatogruppe der allgemeinen Formel -S-S0₃M (mit M der nachstehend genannten Bedeutung) oder die Phosphatogruppe der allgemeinen Formel ―O―PO₃M₂ (mit M der nachstehend genannten Bedeutung) oder vorzugsweise die Sulfatogruppe der allgemeinen Formel -OS0₃M (mit M der nachstehend genannten Bedeutung) bedeutet;
Y ist ein Rest der Formel (3) 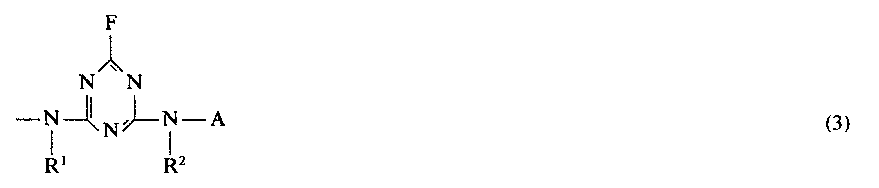 in welcher
R' ein Wasserstoffatom oder eine Alkylgruppe von 1 bis 4 C-Atomen und
R² ein Wasserstoffatom oder eine Alkylgruppe von 1 bis 4 C-Atomen ist,

wobei R¹ und R² zueinander gleiche oder voneinander verschiedene Bedeutungen besitzen können, sowie
A ein Wasserstoffatom oder eine Alkylgruppe von 1 bis 6 C-Atomen ist, die substituiert sein kann, beispielsweise durch einen oder zwei Substituenten aus der Gruppe Methoxy, Äthoxy, Hydroxy, Acetyloxy, Phosphato, Sulfato, Sulfo, Carboxy, Phenyl und Sulfophenyl, oder der Phenylrest ist, der durch Substituenten aus der Gruppe Methyl, Äthyl, Methoxy, Äthoxy, Chlor, Brom, Carboxy, Sulfo, Carbamoyl und Sulfamoyl substituiert sein kann, oder ein Rest der Formel (4) 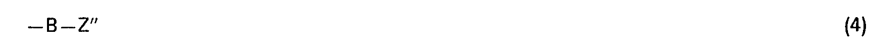 ist, worin
B der Phenylenrest oder ein Naphthylenrest ist, die beide durch einen oder zwei Substituenten substituiert sein können, die aus der Menge von einer Sulfogruppe, einem Chloratom, einer oder zwei Methyl- oder Äthylgruppen und einer oder zwei Methoxy- oder Äthoxygruppen ausgewählt sind, und
Z" eine der für Z genannten Bedeutungen besitzt;
n ist die Zahl oder 2 und
m ist die Zahl Null oder 1,

wobei die Summe von (m + n) gleich 2 ist und
wobei die Verbindung der Formel (1) mindestens drei der im Molekül möglichen Reste entsprechend den oben definierten Formeln (2a) bis (2f) und der nachstehenden Formel (5) 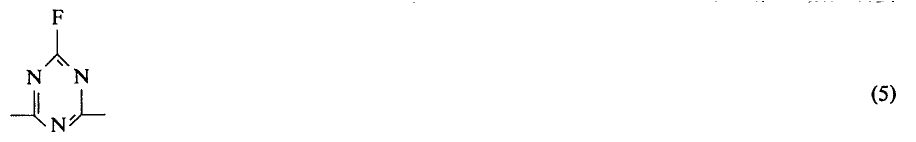 zwingend enthält;
M ist ein Wasserstoffatom oder das Äquivalent- eines Metalls, wie beispielsweise der 1., 2. und 3. Hauptgruppe, vorzugsweise eines ein- oder zweiwertigen Metalls, wie Alkali- und Erdalkalimetalls, insbesondere des Natriums, Kaliums und Calciums.

Diese neuen Verbindungen können in Form der freien Säure und in Form ihrer Salze vorliegen. Bevorzugt sind sie in Form der Salze, insbesondere der Alkali- und Erdalkalimetallsalze, insbesondere bevorzugt als Natrium-, Kalium- und auch Calciumsalze. Sie finden bevorzugt in Form der Alkalimetallsalze Verwendung zum Färben von hydroxy- und/oder carbonamidgruppenhaltigen Materialien, diese vorzugsweise als Fasermaterialien.

Die vorliegende Erfindung betrifft weiterhin ein Verfahren zur Herstellung der neuen Disazoverbindungen der allgemeinen Formel (1), das dadurch gekennzeichnet ist, daß man in äquimolaren Mengen 1-Amino-8-naphthol-4,6-disulfonsäure oder 1-Amino-8-naphthol-3,6-disulfonsäure mit einer Diazoniumverbindung eines Amins der allgemeinen Formel (6) 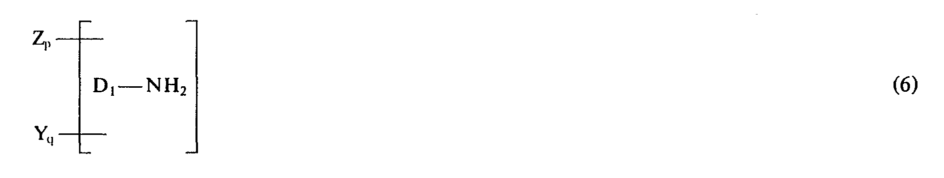 in welcher Dᵢ, Z und Y die obengenannten Bedeutungen haben und p und q jedes für die Zahl Null oder 1 steht, und nachfolgend mit einer Diazoniumverbindung eines Amins der allgemeinen Formel (7) 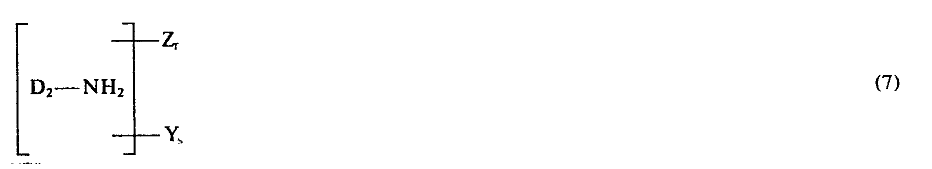 in welcher D_{z}, Z und Y die obengenannte Bedeutung besitzen und r und s jedes für die Zahl Null oder 1 steht, umsetzt und hierbei die erste Diazoniumverbindung in o-Stellung zur Aminogruppe der Aminonaphtholsulfonsäure zur Monoazoverbindung kuppelt und die zweite Diazoniumverbindung in o-Stellung zur Hydroxygruppe der gebildeten Monoazoverbindung einführt, wobei man die Amine der allgemeinen Formeln (6) und (7) so auswählt, daß die Summe von (p+q) gleich 1, die Summe von (r+s) gleich 1, die Summe von (p + r) gleich Null oder 1 und die Summe von (q + s) gleich 1 oder ist.

In den Diazokomponenten der allgemeinen Formeln (6) und (7) können die Formelreste D₁, D_{z}, Z und Y zueinander gleiche oder voneinander verschiedene Bedeutungen besitzen und in ein und derselben Aminoverbindung die Formelreste R¹ und R² jeweils gleiche oder verschiedene Bedeutungen zueinander haben. Weiterhin kann der eventuell in Y vorhandene Rest Z von dem eventuell vorhandenen, an D, oder Dz direkt gebundenen Rest Z verschieden sein. Die zur Herstellung der erfindungsgemäßen Disazoverbindungen in die Reaktion eingesetzten beiden Amine können somit entweder die gleiche oder eine voneinander verschiedene Konstitution haben.

Die Diazotierung der Amine der allgemeinen Formeln (6) und (7) erfolgt in bekannter und üblicher, dem Fachmann geläufiger Weise, beispielsweise mittels salpetriger Säure (Alkalinitrit und eine starke Säure), Nitrosylschwefelsäure oder einem niederen Alkylnitrit.

Die Kupplungsreaktion des diazotierten Amins der allgemeinen Formel (6) oder (7) mit der 1-Amino-8-naphthol-3,6- oder -4,6-disulfonsäure wird zuerst in saurem Medium, wie bei einem pH-Wert zwischen 0 und 4, vorzugsweise zwischen 0,5 und 3, und bspw. bei einer Temperatur zwischen 0 C und 25°C analog bekannten Verfahrensweisen durchgeführt, so daß diese erste Diazoniumverbindung in ortho-Stellung zur Aminogruppe des Aminonaphthols kuppelt; anschließend wird die Kupplung mit der zweiten Diazokomponente in schwach saurem bis schwach alkalischem Medium, wie bei einem pH-Wert zwischen 4 und 8, vorzugsweise zwischen 4,5 und 7,5, und bspw. bei einer Temperatur zwischen 0 und 30°C, analog bekannten Verfahrensweisen durchgeführt, wobei die Kupplung in ortho-Stellung zur Hydroxygruppe des Aminonaphtholrestes der gebildeten Monoazoverbindung erfolgt. Solche Verfahrensweisen sind beispielsweise aus den deutschen Patentschriften 960 534 und 1 644 198 bekannt.

Aromatische Amine der allgemeinen Formel (6) und der allgemeinen Formel (7), in welchen q gleich Null bzw. s gleich Null ist, sind beispielsweise in den deutschen Patentschriften 1 278041, 1 276842, 1150163, 1126542, 1 153029 und in den deutschen Offenlegungsschriften 2 154 943, 2 100 080, 2 034 591, 1 943 904 und in der deutschen Auslegeschrift 1 204 666 beschrieben. Solche aromatischen Amine (6) und (7) sind beispielsweise: Anilin-3-β-sulfatoäthylsufon, Anilin-4-β-sulfatoäthylsulfon, 2-Amino-toluol-4-β-sulfatoäthylsulfon, 2-Amino-anisol-4-β-sulfatoäthylsulfon, 2-Amino-anisol-5-β-sulfatoäthylsulfon, 2,5-Dimethoxy-anilin-4-β-sulfatoäthylsulfon, 2,4-Dimethoxy-anilin-5-β-sulfatoäthylsulfon, 2-Methoxy-5-methyl-anilin-4-β-sulfatoäthylsulfon, 4-Amino-anisol-3-β-sulfatoäthylsulfon, 4-Amino-toluol-3-β-sulfatoäthylsulfon, 4-β-Sulfatoäthylsulfonyl-anilin-2-sulfonsäure, 5-β-Sulfatoäthylsulfonyl-anilin-2-sulfonsäure, 2-Amino-toluol-5-β sulfatoäthylsulfon, 2-Chlor-anilin-4-β sulfatoäthylsulfon, 2-Chloranilin-5-β-sulfatoäthylsulfon, 2-Brom-anilin-4-β-sulfatoäthylsulfon, 2,6-Dichlor-anilin-4-β-sulfatoäthylsulfon, 2,6-Dimethyl-anilin-3-β-sulfatoäthylsulfon, 2,6-Dimethylanilin-4-β-sulfatoäthylsulfon, 2-Naphthylamin-5-β-sulfatoäthylsulfon, 2-Naphthylamin-6-β-sulfatoäthylsulfon, 2-Naphthylamin-8-β-sulfatoäthylsulfon, 8-β-Sulfatoäthylsulfonyl-2-amino-naphthalin-6-sulfonsäure, 6-β-Sulfatoäthylsulfonyl-2-amino-naphthalin-1-sulfonsäure, N-Methyl-N-β-sultatoäthylsulfonyl)-p-phenylendiamin, N-Methyl-N-(β-sulfatoäthylsulfonyl)-m-phenylendiamin, (4-Aminobenzyl)-β-sulfatoäthyl)-sulfon und (3-Aminobenzy))-β-su)fatoäthy))-sutfon sowie deren entsprechenden β-Chloräthyl-, β-Acetoxy-äthyl-, β-Phosphatoäthyl-"d Thiosulfatoäthyl- und Vinylsulfonyl-Derivate.

Aromatische Amine der allgemeinen Formel (6) und der allgemeinen Formel (7), in welchen p gleich Null bzw. r gleich Null ist, lassen sich beispielsweise herstellen, indem man ein Diamin der allgemeinen Formel R^{l-}NH-Di-NH₂ oder R¹―NH―D₂―NH₂, in welchen R¹, Di und Dz die obengenannten Bedeutungen haben, mit 2,4,6-Trifluor-s-triazin (Cyanurfluorid), beispielsweise bei ―5°C bis +5°C und einem pH-Wert zwischen 6 und 7, umsetzt und das so gebildete Produkt entsprechend der allgemeinen Formel (8a) oder (8b) 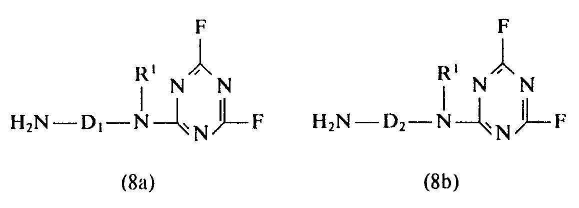 in welchen Dᵢ, D₂ und R¹ die obengenannten Bedeutungen haben, beispielsweise bei 10-30°C und einem pH-Wert zwischen 6 und 7, mit einem Amin der allgemeinen Formel R²NH―A, in welcher R² und A die obengenannten Bedeutungen besitzen, zu einem Amin (6) bzw. (7) umsetzt, oder indem man unter Umkehrung der Reihenfolge dieser beiden Reaktionsschritte zuerst das obengenannte und definierte Amin der allgemeinen Formel R^{z}NH―A mit Cyanurfluorid, beispielsweise bei 0°C bis 5°C und einem pH-Wert zwischen 6 und 7, zu einer Verbindung der allgemeinen Formel (9) 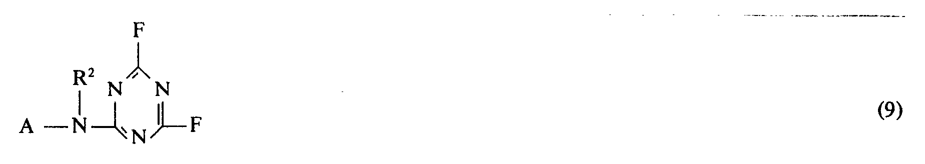 reagieren läßt und sodann diese Verbindung (9) im zweiten Reaktionsschritt, beispielsweise bei 20―25° C und einem pH-Wert zwischen 6 und 7, mit einem obengenannten und definierten Diamin der allgemeinen Formel R¹―NH―D₁―NH₂ oder R¹―NH―D₂―NH₂ umsetzt. Solche aromatischen Amine (6) oder (7) mit p gleich Null bzw. r gleich Null, die nach den obengenannten Verfahrensweisen hergestellt werden können, sind Verbindungen entsprechend der allgemeinen Formel (10a) oder (10b) 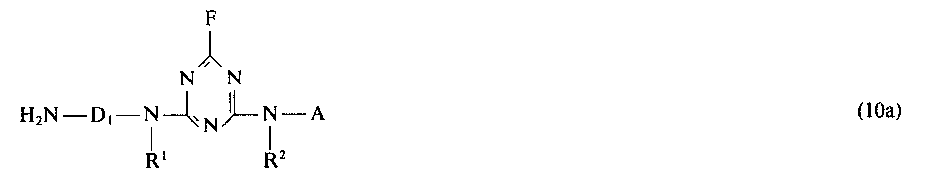 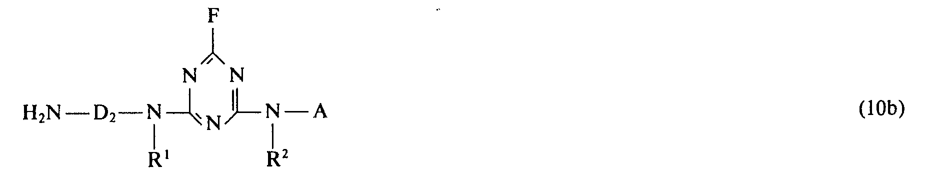 in welchen D₁, D₂, R¹, R² und A die obengenannten Bedeutungen haben.

In Formel (10a) kann der Rest H₂N―D₁ beispielsweise den 3-Amino-4-sulfophenyl-, den 4-Amino-3-sulfo-phenyl-, 4-Amino-2,5-disulfo-phenyl-, 3-Amino-4,6-disulfo-phenyl-, 4-Amino-phenyl-, 3-Amino-2-methyl-5-sulfo-phenyl-, 3-Amino-4-chlor-phenyl-, 4-Amino-3,5-disulfo-phenyl-, 3-Amino-4-methyl-phenyl-, 3-Amino-4-methoxy-phenyl-, 6-Amino-4,8-disulfo-naphth-2-yl-, den 5-Amino-3,7-disulfo- naphth-1-yl- oder den 3-Amino-phenyl-Rest bedeuten

In Formel (10b) kann der Rest H₂N―D₂― beispielsweise den 3 Amino-4-sulfo-phenyl-, den 4-Amino-3-sulfo-phenyl- oder den 4-Amino-phenylrest bedeuten.

Bevorzugt stellen die Formelglieder R¹ und R² in Formel (10a) jedes ein Wasserstoffatom dar. In Formel (10b) sind die Formelglieder R¹ und R² bevorzugt jedes ein Wasserstoffatom oder eine Methylgruppe; bevorzugt ist der Rest H₂N - D₂ - der 3-Amino-4-sulfo-phenyl- oder der 4-Amino-3-sulfo-phenyl-Rest, wenn R¹ ein Wasserstoffatom und R² eine Methylgruppe darstellen, oder bevorzugt der 4-Amino-phenyl-Rest, wenn R¹ eine Methylgruppe und R² ein Wasserstoffatom darstellen.

In den Formeln (10a) und (10b) kann das Formelglied A ebenfalls jeweils einen Rest der allgemeinen Formel (4a) 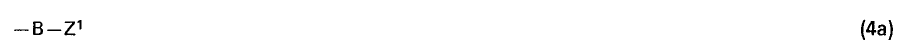 darstellen, in welchen B die obengenannte Bedeutung besitzt und Z¹ für die β-Chloräthylsulfonyl, β-Acetoxyäthylsulfonyl-, β-Thiosulfataäthylsulfonyl-, β-Phosphatoäthylsulfonyl- oder die Vinylsulfonyl-Gruppe oder bevorzugt für die-Sulfatoäthylsulfonyl-Gruppe steht. Der Formelrest B in Formel (4) oder (4a) ist beispielsweise der 1,4-Phenylen-, 1,3-Phenylen-, 4-Methyl-1,3-phenylen-, 4-Methoxy-1,3-phenylen-, 3-Methoxy-1,4-phenylen-, 2,5-Dimethoxy-1,4-phenylen-, 4,6-Dimethoxy-1,3-phenylen-, 2-Methyl-5-methoxy-1,4-phenylen-, 3-Chlor-1,4-phenylen-, 6-Methoxy-1,3-phenylen-, 6-Methyl-1,3-phenylen-, 4-Sulfo-1,3-phenylen-, 4-Methyl-1,3-phenylen-, 4-Chlor-1,3-phenylen-, 3-Brom-1,4-phenylen- oder der 2,4-Dimethyl-1,3-phenylen-Rest ist, wobei die oben definierte Gruppe Z¹ jeweils in 1-Stellung der Phenylenreste gebunden ist.

Der Formelrest A kann ebenfalls entsprechend der allgemeinen Formel (4) bzw. (4a) beispielsweise einen der nachstehend formelmäßig angegebenen Reste 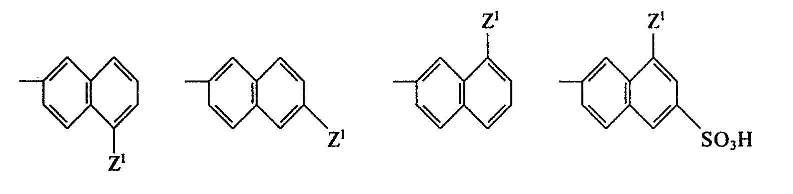 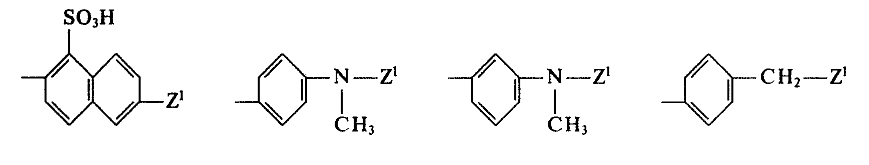 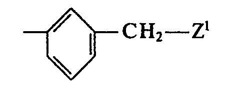 bedeuten, in welchen Z^{l} eine der obengenannten Bedeutungen besitzt.

Verbindungen entsprechend der allgemeinen Formel (1), die eine oder zwei dieser Diazokomponenten (10a) und (10b) enthalten, sind bevorzugt.

Bevorzugt sind insbesondere die erfindungsgemäßen Verbindungen entsprechend den allgemeinen Formeln (11), (12), (13), (14), (15) und (16): 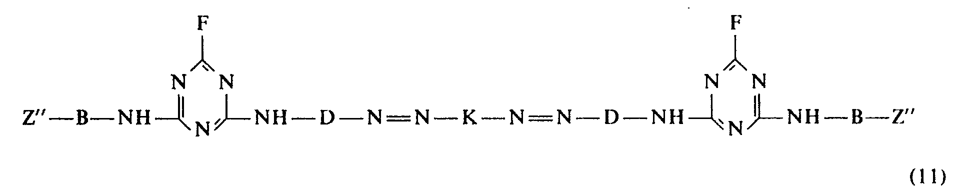 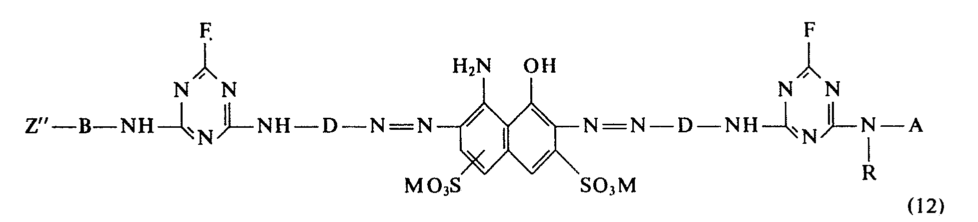 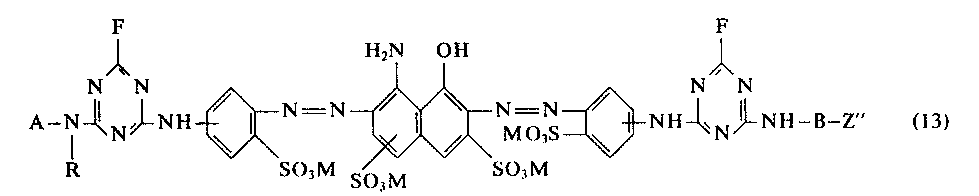 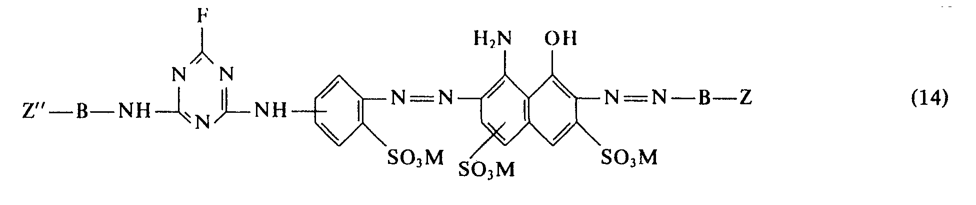 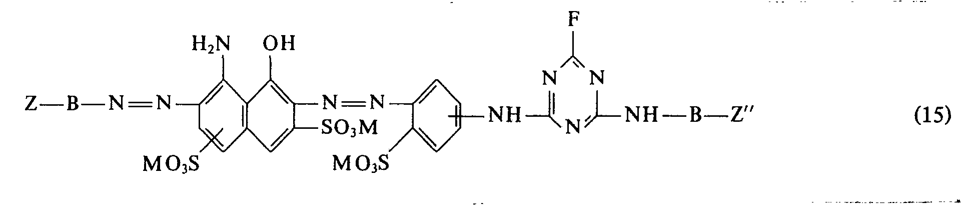 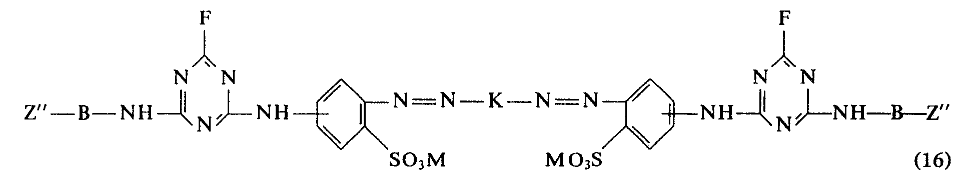 In diesen Formeln bedeuten:
Z und Z" haben die anfangs, insbesondere für Z¹ genannten Bedeutungen und sind insbesondere bevorzugt die β-Sulfatoäthylsulfonyl- oder Vinylsulfonyl-Gruppe;
B ist der Phenylenrest, der durch 1 Sulfogruppe oder durch 1 Methylgruppe oder Methoxygruppe oder 1 Chloratom oder 2 Methoxygruppen oder 1 Methoxygruppe und 1 Methylgruppe substituiert sein kann, wobei die beiden Formelreste B gleich oder verschieden voneinander sein können;
D ist der Phenylenrest, der durch 1 oder 2 Sulfogruppen oder 1 Methoxygruppe, 1 Methylgruppe oder 1 Chloratom oder durch 2 Methoxygruppen oder durch 1 Methoxygruppe und eine Methylgruppe substituiert sein kann, wobei die beiden Formelreste D gleich oder verschieden voneinander sein können;
A ist eine Methyl- oder Äthylgruppe, eine β-Hydroxyäthyl-, β-Sulfoäthyl-, Carboxyäthyl-, ⱼl-Sulfatö- äthyl- oder Sulfophenylgruppe;
R ist ein Wasserstoffatom, eine Methylgruppe oder eine Äthylgruppe;
K steht für den bivalenten Rest der als Kupplungskomponente dienenden doppel-ankuppelbaren 1-Amino-8-naphthol-3,6- oder -4,6-disulfonsäure;
M hat die obengenannte Bedeutung;

die beiden freistehenden Aminogruppen in den Formeln (13) bis (16) sind in meta- oder para-Stellung zu den Azogruppen gebunden.

Die Abscheidung bzw. Isolierung der erfindungsgemäß hergestellten Verbindungen der allgemeinen Formel (1) erfolgt nach allgemein bekannten Methoden entweder durch Ausfällen aus dem Reaktionsmedium mittels Elektrolyten, wie beispielsweise Natriumchlorid oder Kaliumchlorid, oder aber durch Eindampfen der Reaktionslösung, beispielsweise durch Sprühtrocknung. In manchen Fällen kann es auch wünschenswert sein, die Farbstofflösung, gegebenenfalls nach Zusatz von Puffersubstanzen und gegebenenfalls nach eventuellem Konzentrieren, direkt als Flüssigpräparation der färberischen Verwendung zuzuführen.

Die erfindungsgemäßen Disazoverbindungen der allgemeinen Formel (1) besitzen wertvolle Farbstoffeigenschaften, die gleichzeitig infolge ihrer Fluortriazinylen-Komponente und des Restes Z faserreaktive Eigenschaften aufweisen. Die neuen Verbindungen werden bevorzugt zum Färben (im allgemeinen Sinne) von hydroxy-, amino- oder carbonamidgruppenhaltigen Materialien, beispielsweise in Form von Flächengebilden, wie Papier und Leder, oder in der Masse, wie Polyamid und Polyurethan, insbesondere von solchen Materialien in Faserform, verwendet.

In der FR-A-2 437 426 sind zwar Disazofarbstoffe mit der 1-Amino-8-naphthol-3,6-disulfonsäure als bivalenter Kupplungskomponente mit einem (β-Sulfatoäthylsulfonyl-phenylamino)-fluor-s-triazinyla- mino-Reaktivrest bekannt, jedoch wird an keiner Stelle dieser FR-A-2 437 426 ein Typ der Gattung von Disazoverbindungen der vorliegenden Erfindung beschrieben. Der vorliegende, beanspruchte Erfindungsgegenstand ist deshalb neu. Er ist aber auch erfinderisch, wie sich aus den nicht vorhersehbaren und als überraschend zu erachtenden Effekten von durchgeführten Versuchen ergibt.

Die vorliegende Erfindung betrifft somit auch die Verwendung der Verbindungen der allgemeinen Formel (1) zum Färben (einschließlich Massefärbung und Druckfärbung) dieser Materialien bzw. Verfahren zum Färben solcher Materialien in an und für sich üblicher Verfahrensweise, bei welchen eine Verbindung der allgemeinen Formel (1) als Farbmittel eingesetzt wird. Bevorzugt kommen die Materialien in Form von Fasermaterialien zurAnwendung, insbesondere in Form von Textilfasern. Hydroxygruppenhaltige Materialien sind natürliche oder synthetische hydroxygruppenhaltige Ma terialien, wie beispielsweise Cellulosefasermaterialien oder deren Regeneratprodukte oder Polyvinylalkohole. Celulosefasermaterialien sind vorzugsweise Baumwolle, aber auch andere Pflanzenfasern, wie Leinen, Hanf, Jute und Ramiefasern oder regenerierte Cellulosefasern. wie beispielsweise Zellwolle und Viskosekunstseide.

Carbonamidgruppenhaltige Materialien sind beispielsweise synthetische und natürliche Polyamide und Polyurethane, insbesondere in Form der Fasern, beispielsweise Wolle und andere Tierhaare, Seide, Leder, Polyamid-6,6, Polyamid-6, Polyamid-11 und Polyamid-4.

Die erfindungsgemäßen Farbstoffe lassen sich auf den genannten Substraten nach den für Reaktivfarbstoffe bekannten Anwendungstechniken applizieren. So erhält man mit ihnen auf Cellulosefasern nach dem Ausziehverfahren aus langer Flotte zum Beispiel unter Verwendung der verschiedensten Alkalizusätze sehr gute Farbausbeuten.

Nach den Klotzverfahren werden auf Cellulosefasern ebenfalls ausgezeichnete Farbausbeuten erhalten, wobei durch Verweilen bei Raumtemperatur, durch Dämpfen oder mit Trockenhitze fixiert werden kann.

Nach den üblichen Druckverfahren für Cellulosefasern, einphasig in Anwesenheit von Natriumbicarbonat oder anderer säurebindender Mittel in der Druckpaste und anschließendem Dämpfen bei 100-103° C oder zweiphasig mit neutraler oder schwach saurer Druckpaste bedruckt und dann entweder durch ein heißes elektrolythaltiges alkalisches Bad geführt oder aber mit einer alkalischen elektrolythaltigen Klotzflotte überklotzt und dann durch Verweilen, Dämpfen oder Trockenhitze entwickelt, erhält man ebenfalls farbstarke Drucke mit gutem Stand der Konturen und einem klaren Weißfond. Der Ausfall der Drucke ist von wechselnden Fixierbedingungen nur wenig abhängig. Sowohl in der Färberei als auch in der Druckerei sind die mit den erfindungsgemäßen Farbstoffen erhaltenen Fixiergrade außergewöhnlich hoch.

Die Echtheiten der auf Cellulosefasern mit Hilfe der erfindungsgemäßen Farbstoffe erhaltenen Färbungen und Drucke sind beachtlich. Dies gilt sowohl für die wichtigsten Fabrikations- als auch für die wichtigsten Gebrauchsechtheiten. Besonders zu erwähnen sind die Lichtechtheit, die Naßechtheiten, wie Waschechtheiten, Walkechtheiten, Wasserechtheit, Seewasserechtheit, Überfärbeechtheit, Schweißechtheit, sowie Plissierechtheit, Bügelechtheit und Reibechtheit.

Die Färbungen auf Polyamidfasern werden üblicherweise aus saurem Milieu ausgeführt. So kann man beispielsweise dem Färbebad Essigsäure oder Essigsäure und Ammoniumacetat zufügen, um den gewünschten pH-Wert zu erhalten. Zwecks Erreichung einer brauchbaren Egalität der Färbungen empfiehlt sich ein Zusatz an üblichen Egalisiermitteln, beispielsweise auf Basis eines Umsetzungsproduktes von Cyanurchlorid mit der dreifach molaren Menge einer Aminobenzol- und/oder einer Aminonaphthalinsulfonsäure und/oder auf Basis eines Umsetzungsproduktes von beispielsweise Stearylamin mit Äthylenoxyd. Die Färbungen können sowohl bei Siedetemperatur als auch bei 110-120°C ausgeführt werden.

Die nachstehenden Beispiele dienen zur Erläuterung der Erfindung. Die darin genannten Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die formelmäßig beschriebenen erfindungsgemäßen Verbindungen sind in der Regel in Form der freien Säuren angegeben; im allgemeinen werden sie in Form ihrer Salze, wie Kalium- oder Natriumsalze, hergestellt und isoliert und in Form ihrer Salze zum Färben eingesetzt. Ebenso sind die insbesondere in den Tabellenbeispielen formelmäßig beschriebenen Ausgangsverbindungen in der Regel in Form der freien Säure angegeben; sie können sowohl in saurer Form als auch in Form ihrer Salze, wie Natrium- oder Kaliumsalze, in die Synthese der erfindungsgemäßen Disazoverbindungen eingesetzt werden.

### Beispiel 1

Zu einer neutralen Lösung von 28,1 Teilen Anilin-4-,8-sulfato-äthylsulfon in 150 Teilen Wasser werden bei 0°C innerhalb von 15 Minuten 13,7 Teile Cyanurfluorid zugetropft, wobei zur Einhaltung des pH-Wertes bei 6,0 wäßriges Natriumhydroxid zugegeben wird. Anschließend wird zum erhaltenen Reaktionsgemisch eine neutrale Lösung von 26,8 Teilen 1,4-Diamino-benzol-2,5-disulfonsäure in 200 Teilen Wasser zugefügt und das so gewonnene Gemisch bei 18 bis 20 C und einem pH-Wert von ca. 6,0 bis 7,0 einige Stunden gerührt, wobei der pH-Wert durch portionsweise Zugabe von wäßrigen Natriumhydroxid in dem angegebenen Bereich gehalten wird. Das gebildete Reaktionsprodukt liegt dann in einer klaren Lösung vor.

In diese Lösung werden 250 Teile Eis und anschließend 60 Volumenteile einer 310/oigen wäßrigen Salzsäure gegeben; die anschließende Diazotierung erfolgt durch Zutropfen von 20 Volumenteilen einer wäßrigen 5 n-Natriumnitritlösung bei einer Temperatur von 0 bis 5° C. Die erhaltene Diazoniumsalzlösung wird sodann bei einem pH-Wert von 2,5 bis 3,0 und einer Temperatur von 0 bis 10° C in eine Lösung von 31,9 Teilen 1-Amino-8-naphthol-3,6-disulfonsäure in 1500 Teilen Wasser eingetropft, wobei der pH durch portionsweise Zugabe von 63 Teilen kristallisiertem Natriumacetat bei diesem Wert von 2,5 bis 3,0 gehalten wird. Nach beendeter Kupplung wird die gebildete Monoazoverbindung mittels Kaliumchlorid ausgefüllt und abgesaugt.

Der feuchte Filterkuchen dieser Monoazoverbindung wird in 500 Teilen Wasser gelöst. Ein weiterer Ansatz des wie oben beschriebenen diazotierten sekundären Kondensationsproduktes aus Cyanurfluorid und Anilin-4-,fl-sulfatoäthylsulfon sowie 1,4-Diaminobenzol-2,5-disulfonsäure wird in diese wäßrige Lösung der Monoazoverbindung eingetropft, wobei die Temperatur bei 10 bis 18°C und der pH-Wert bei 6,0 bis 6,5 gehalten werden. Die Kupplungsreaktion wird noch 4 Stunden unter Rühren weitergeführt. Die Lösung wird anschließend geklärt und die gebildete Disazoverbindung durch Zugabe von Natriumchlorid ausgesalzen. Diese wird sodann abgesaugt, bei 40 bis 50° C getrocknet und gemahlen.

Es wird ein schwarzes, elektrolythaltiges (vorwiegend Natriumchlorid) Pulver erhalten, welches das Alkalimetallsalz, vorwiegend Natriumsalz, der Verbindung der Formel 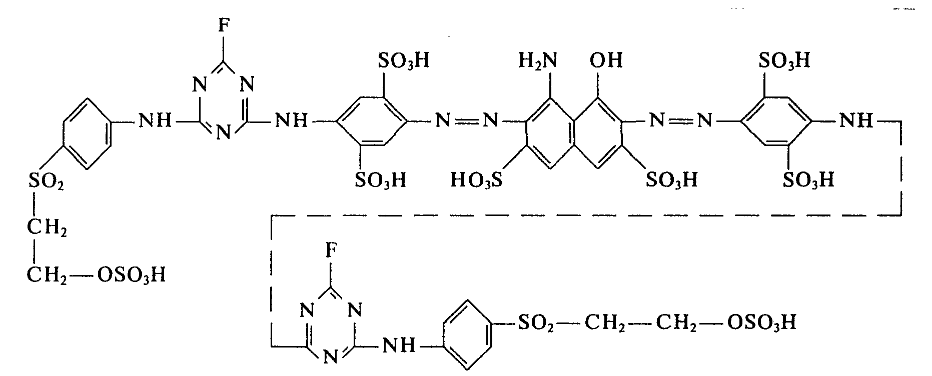 enthält. Diese zeigt sehr gute Farbstoffeigenschaften und färbt nach den für Reaktivfarbstoffe üblichen Färbe- und Druckmethoden und Fixierverfahren Cellulosefasermaterialien, wie Baumwolle, in grünstichig schwarzen Farbtönen; diese Färbungen und Drucke zeigen sehr gute Gebrauchs- und Fabrikationsechtheiten, wie insbesondere eine sehr gute Wasch-, Schweiß-, Reib-, Wasser-, Säure-und Alkaliechtheit. Bei Druckverfahren auf Cellulosefasern ist die Dampfbeständigkeit und das gute Verhalten beim Auswaschen neben dem hohen Fixiergrad und gleichem Ausfall der nach verschiedenen Verfahren fixierten Drucke besonders zu erwähnen.

### Beispiele 2 bis 42

Verfährt man in einer der erfindungsgemäßen Verfahrensweisen zur Herstellung der erfindungsgemäßen Verbindungen entsprechend der allgemeinen Formel (1), so beispielsweise analog der im Beispiel 1 angegebenen Verfahrensweise, und setzt hierzu als Ausgangsverbindungen in entsprechender Weise 1,3- oder 1,4-Diaminobenzol-2-sulfonsäure, Cyanurfluorid und ein Anilin- oder Naphthylaminderivat mit einer_{/}l Sulfatoäthylsulfonylgruppe, welche aus den nachfolgenden Tabellenbeispielen in Verbindung mit der allgemeinen Formel (A) ersichtlich sind, als Kondensationskomponenten sowie 1-Amino-8-naphthol-3,6- oder -4,6-disulfonsäure als Kupplungskomponenten ein, so erhält man die in diesen Tabellenbeispielen angegebenen erfindungsgemäßen Disazoverbindungen, die sehr gute Farbstoffeigenschaften besitzen und ebenfalls auf naürlichen und synthetischen Polyamidfasermaterialien, insbesondere jedoch auf natürlichen und regenerierten Cellulosefasermaterialien, wie insbesondere Baumwolle, insbesondere nach den für faserreaktive Farbstoffe üblichen Färbe- und Druckmethoden und Fixierweisen, farbstarke Färbungen und Drucke mit sehr guten Echtheitseigenschaften und den in den Tabellenbeispielen angegebenen Farbtönen liefern.

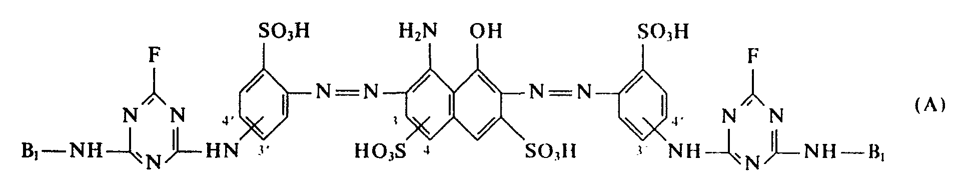

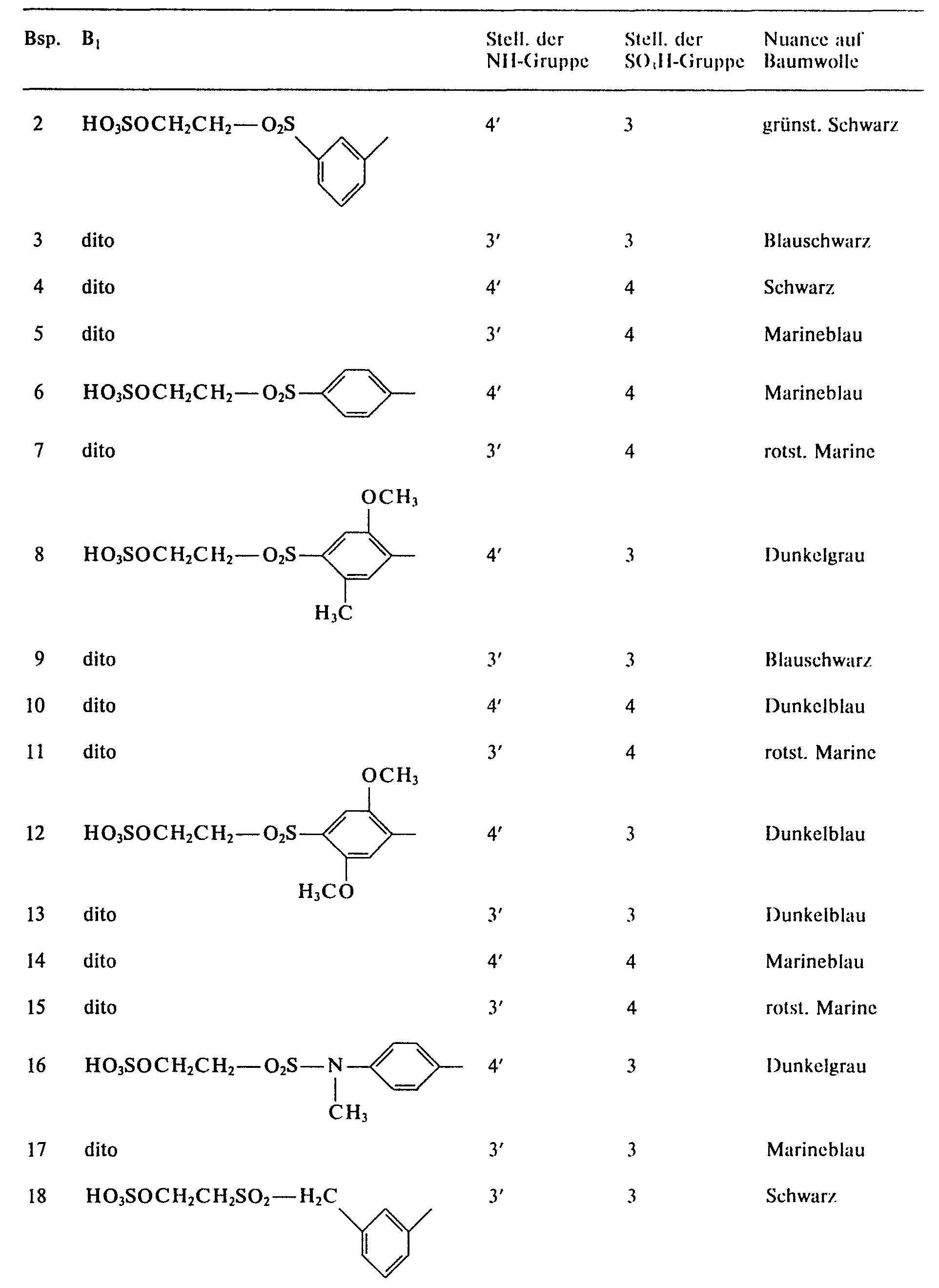

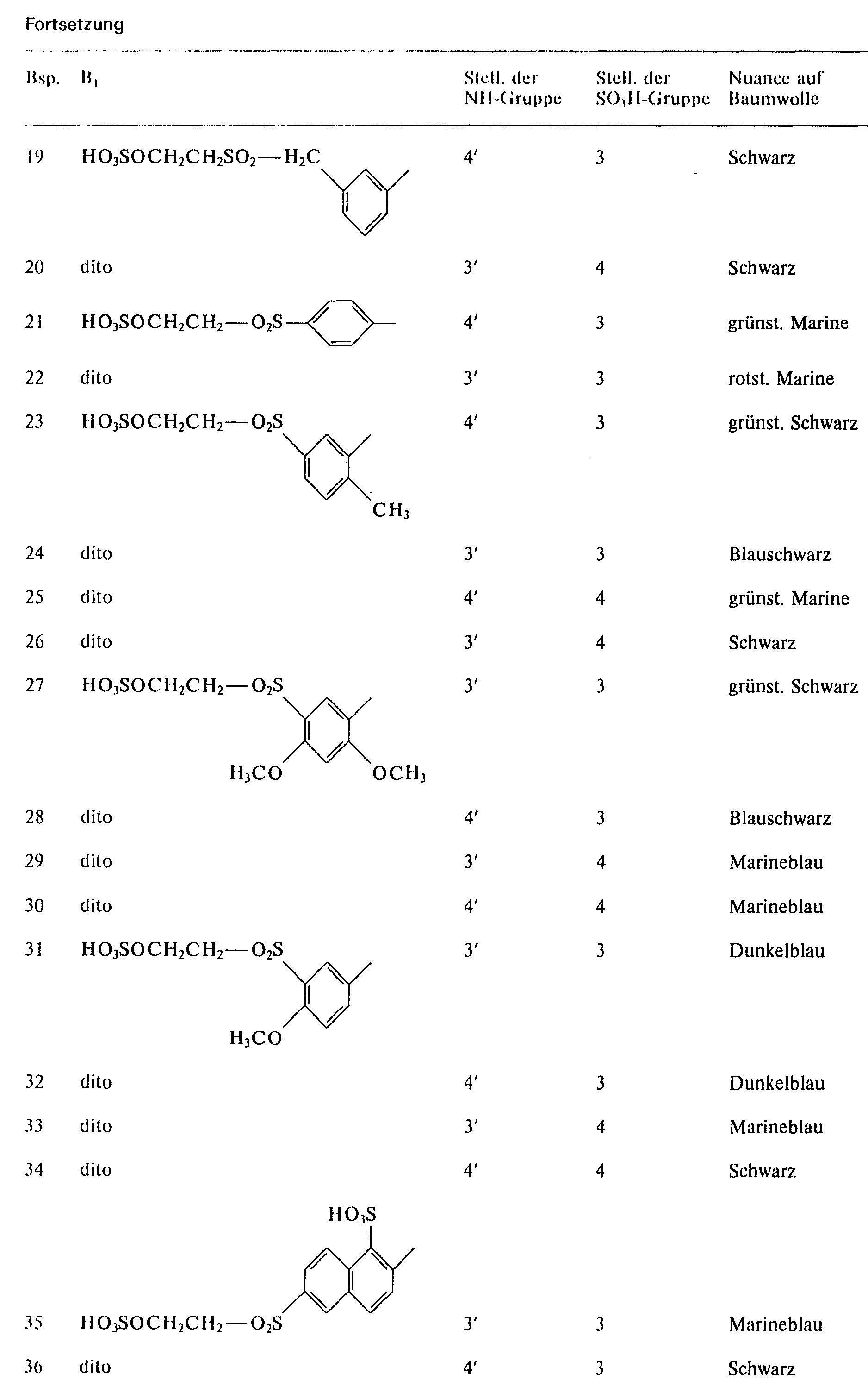

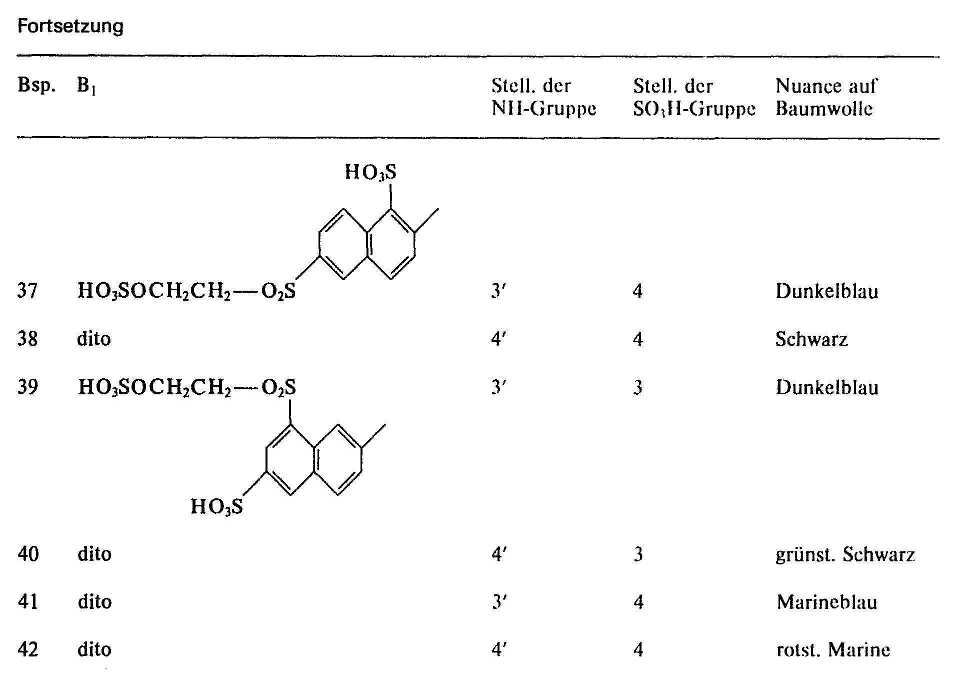

### Beispiele 43 bis 229

Verfährt man in einer der erfindungsgemäßen Verfahrensweisen zur Herstellung der erfindungsgemäßen Verbindungen entsprechend der allgemeinen Formel (1), so beispielsweise analog der im Beispiel 1 angegebenen Verfahrensweise, und setzt hierzu als Ausgangsverbindungen in entsprechender Weise 1,3- oder 1,4-Diaminobenzol-Derivate oder 1,5- oder 2,6-Diaminonaphthalin-Derivate, Cyanurfluorid und ein Anilin- oder Naphthylaminderivat mit einer β-Sulfatoäthylsulfonylgruppe, welche aus den nachfolgenden Tabellenbeispielen in Verbindung mit der allgemeinen Formel (B) ersichtlich sind, als Kondensationskomponenten sowie 1-Amino-8-naphthol-3,6- oder -4,6-disulfonsäure als Kupplungskomponenten ein, so erhält man die in diesen Tabellenbeispielen angegebenen erfindungsgemäßen Disazoverbindungen, die sehr gute Farbstoffeigenschaften besitzen und ebenfalls auf natürlichen und synthetischen Polyamidfasermaterialien, insbesondere jedoch auf natürlichen und regenerierten Cellulosefasermaterialien, wie insbesondere Baumwolle, insbesondere nach den für faserreaktive Farbstoffe üblichen Färbe- und Druckmethoden und Fixierweisen farbstarke Färbungen und Drucke mit sehr guten Echtheitseigenschaften und den in den Tabellenbeispielen angegebenen Farbtönen liefern.

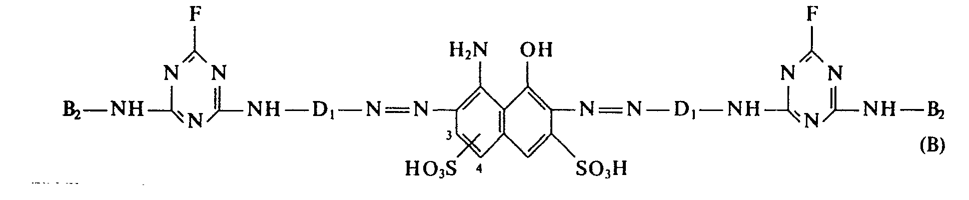

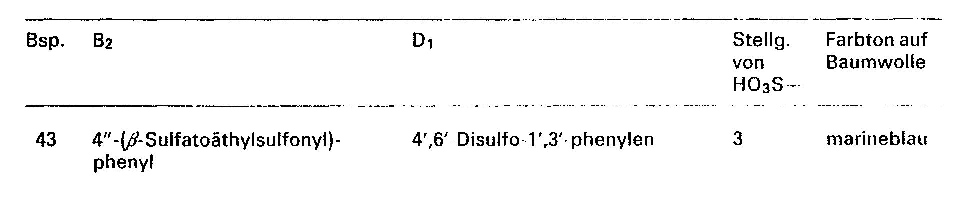

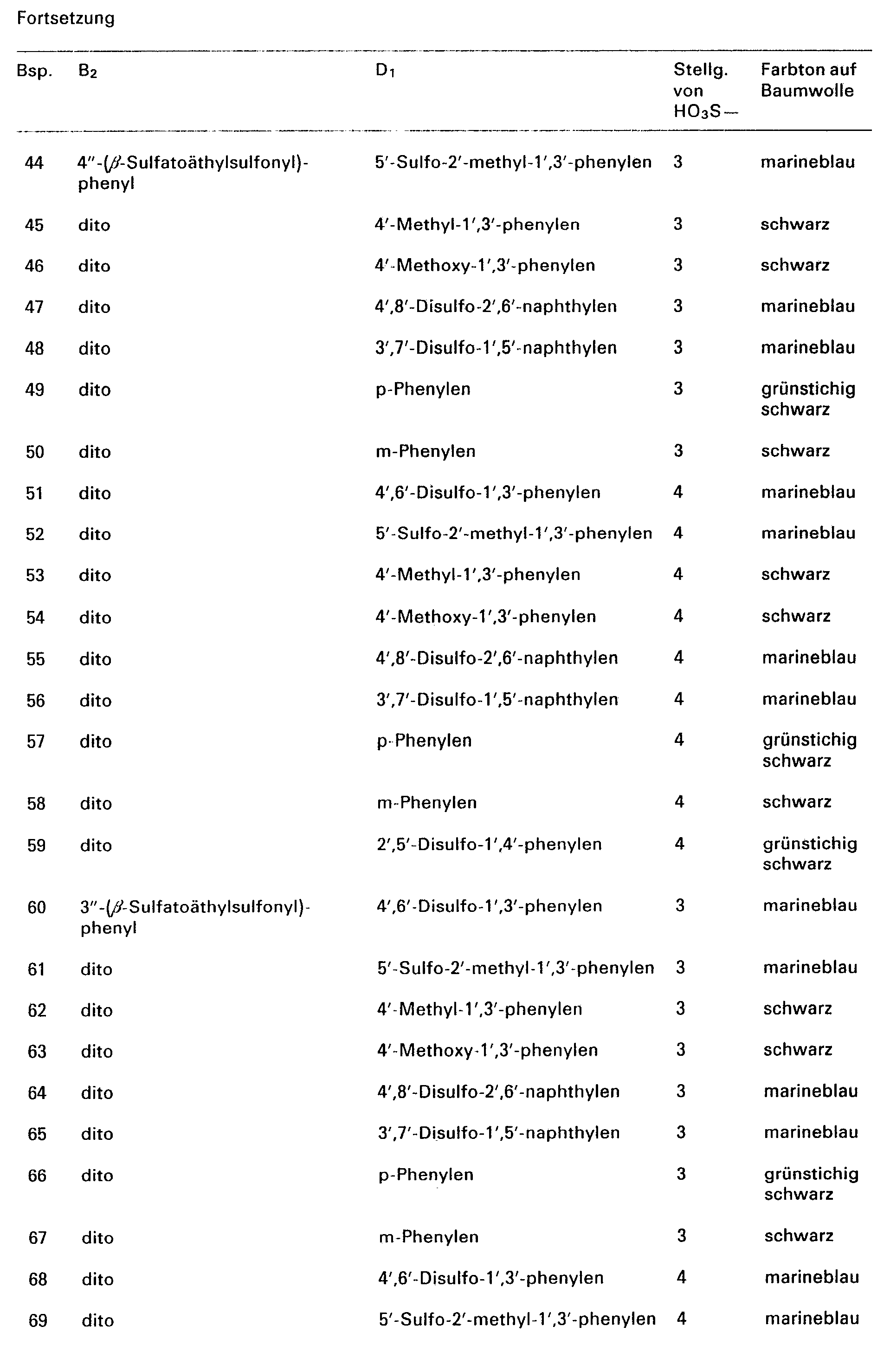

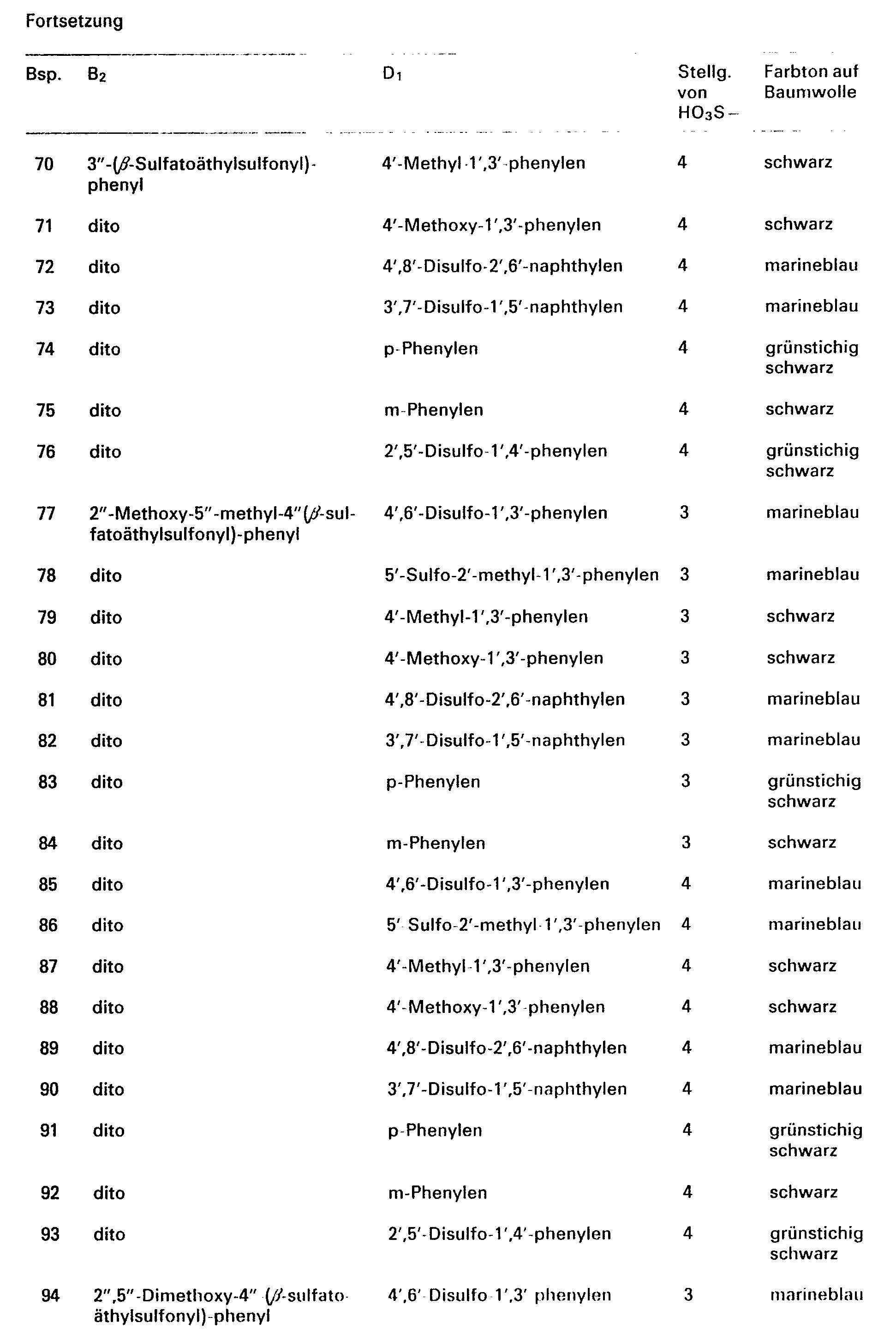

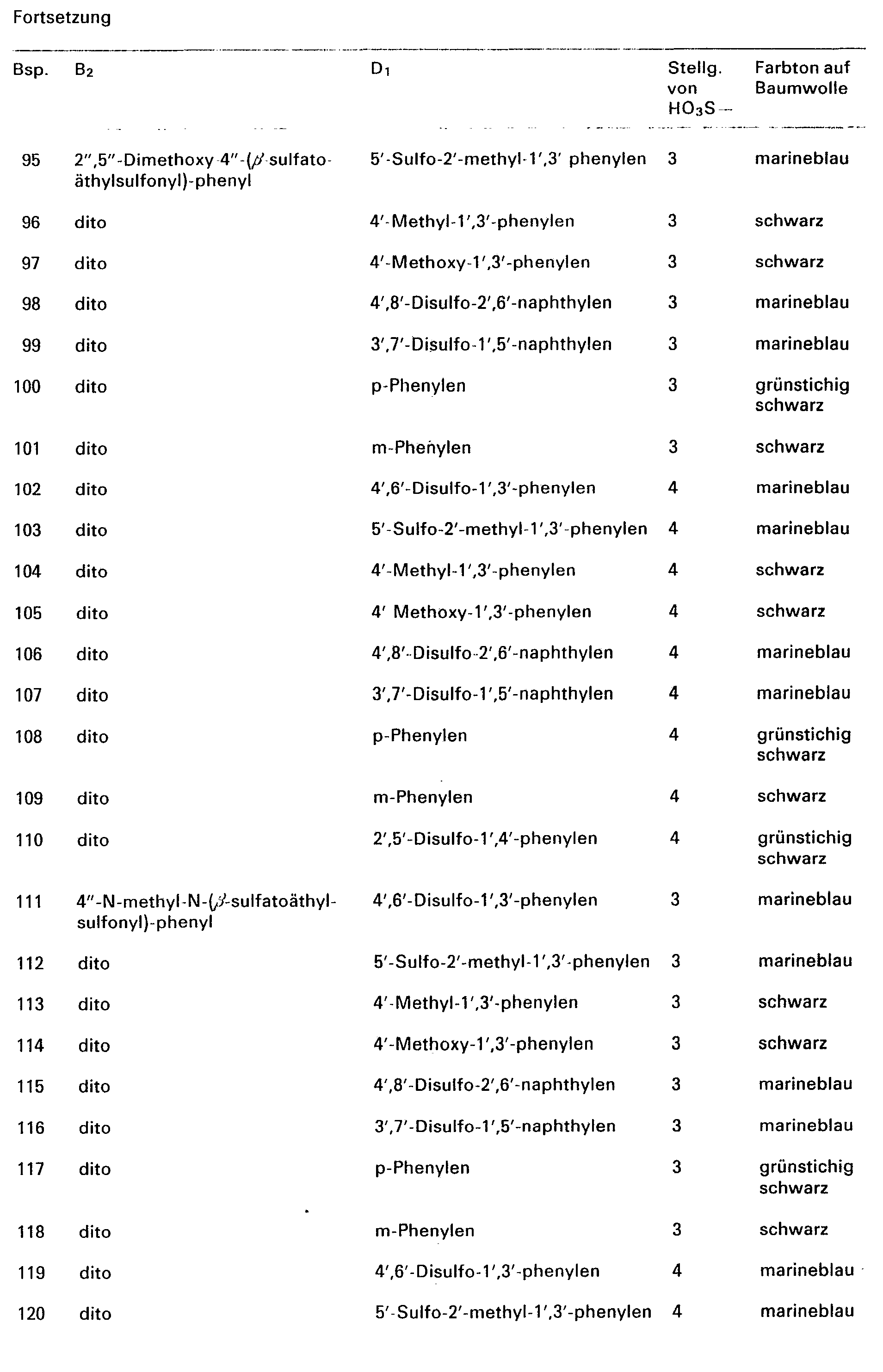

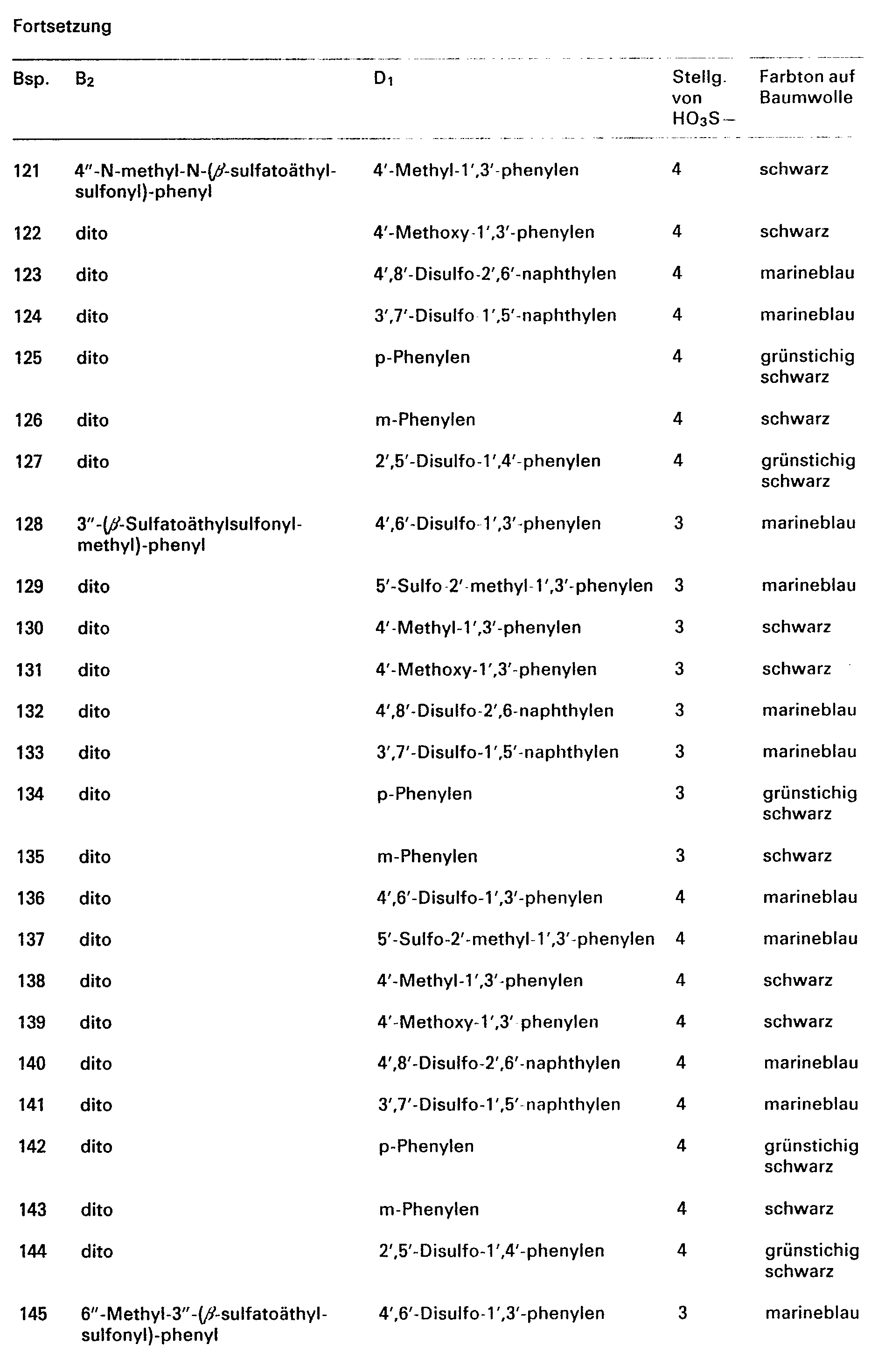

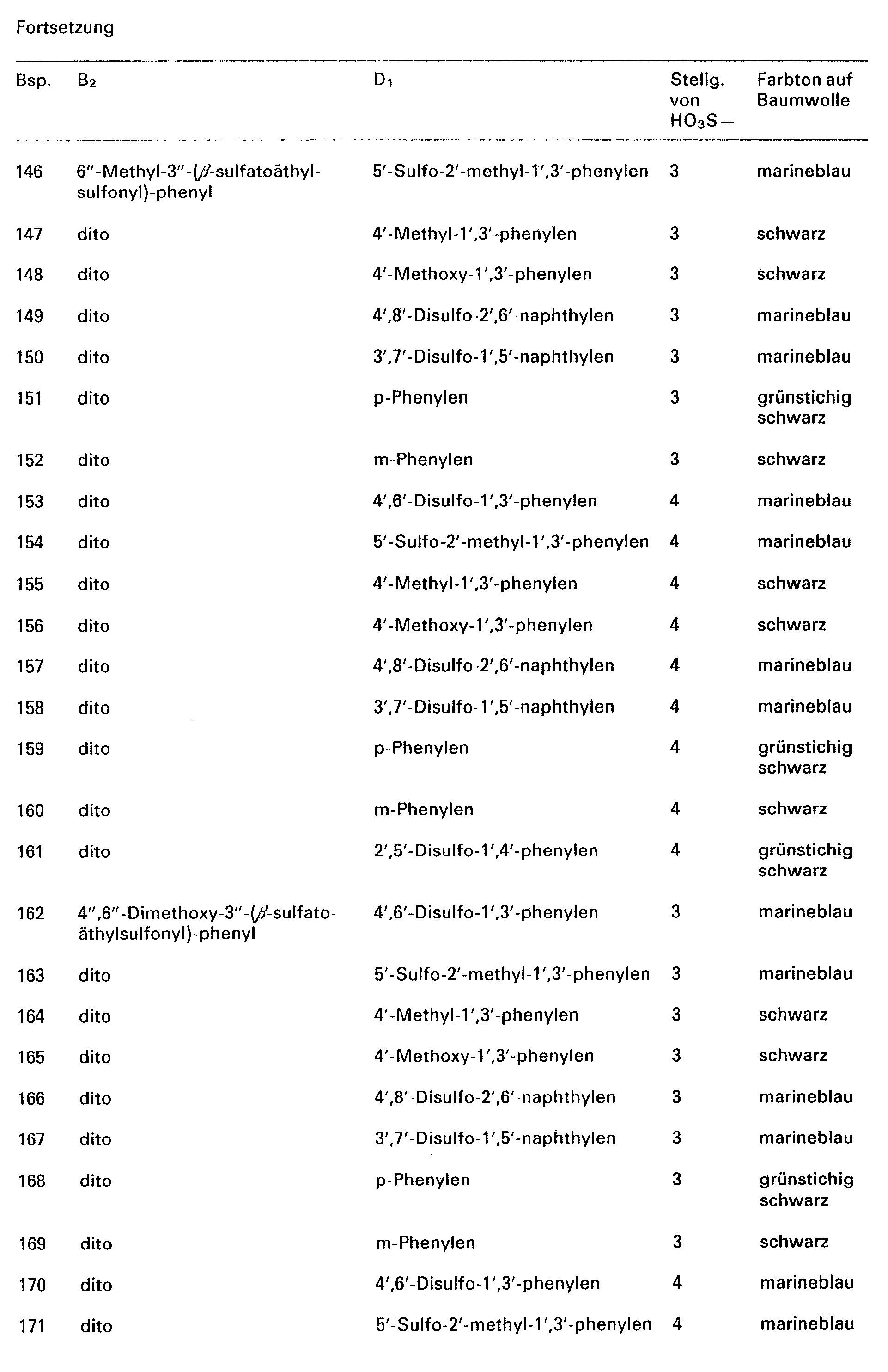

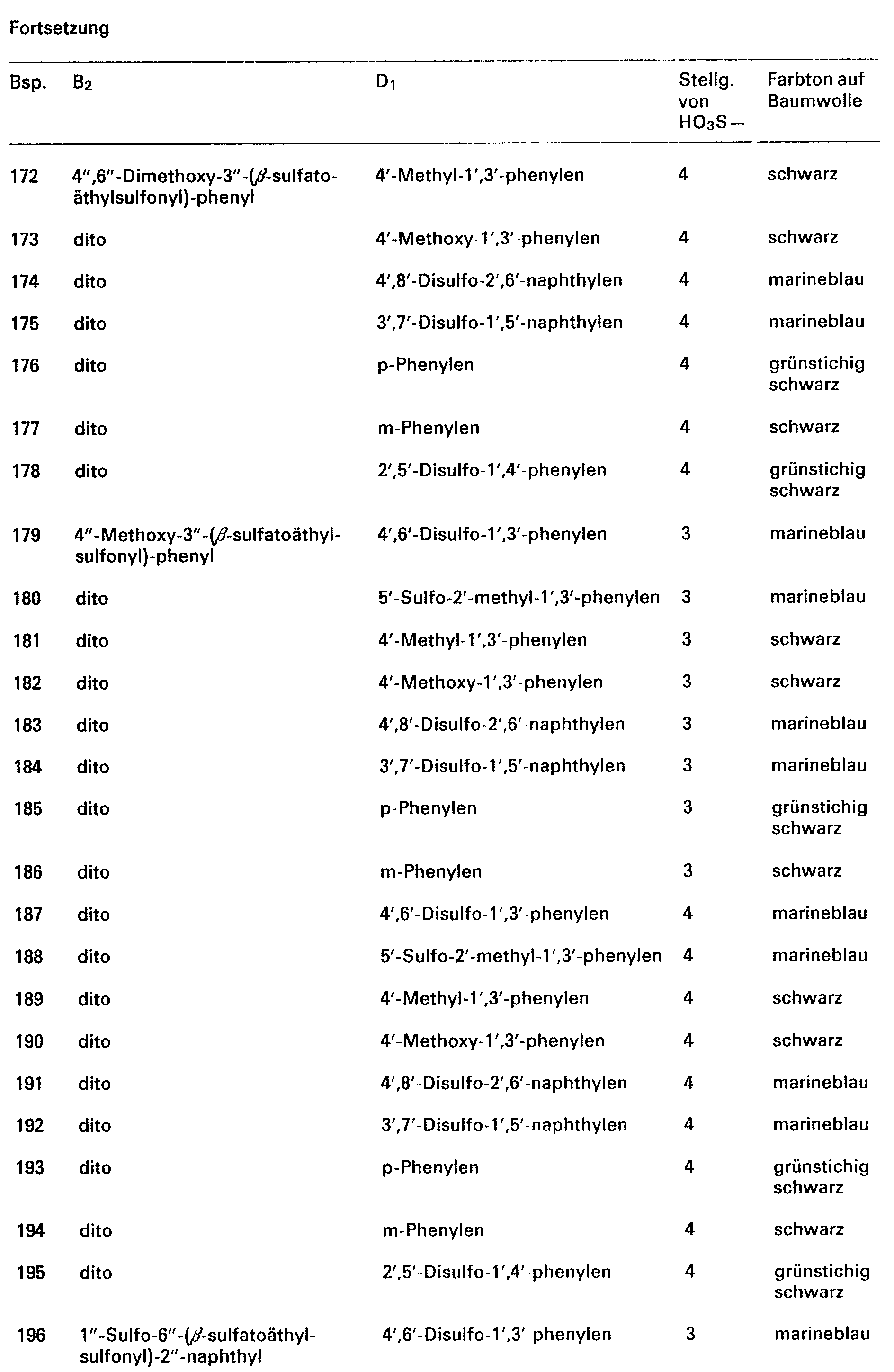

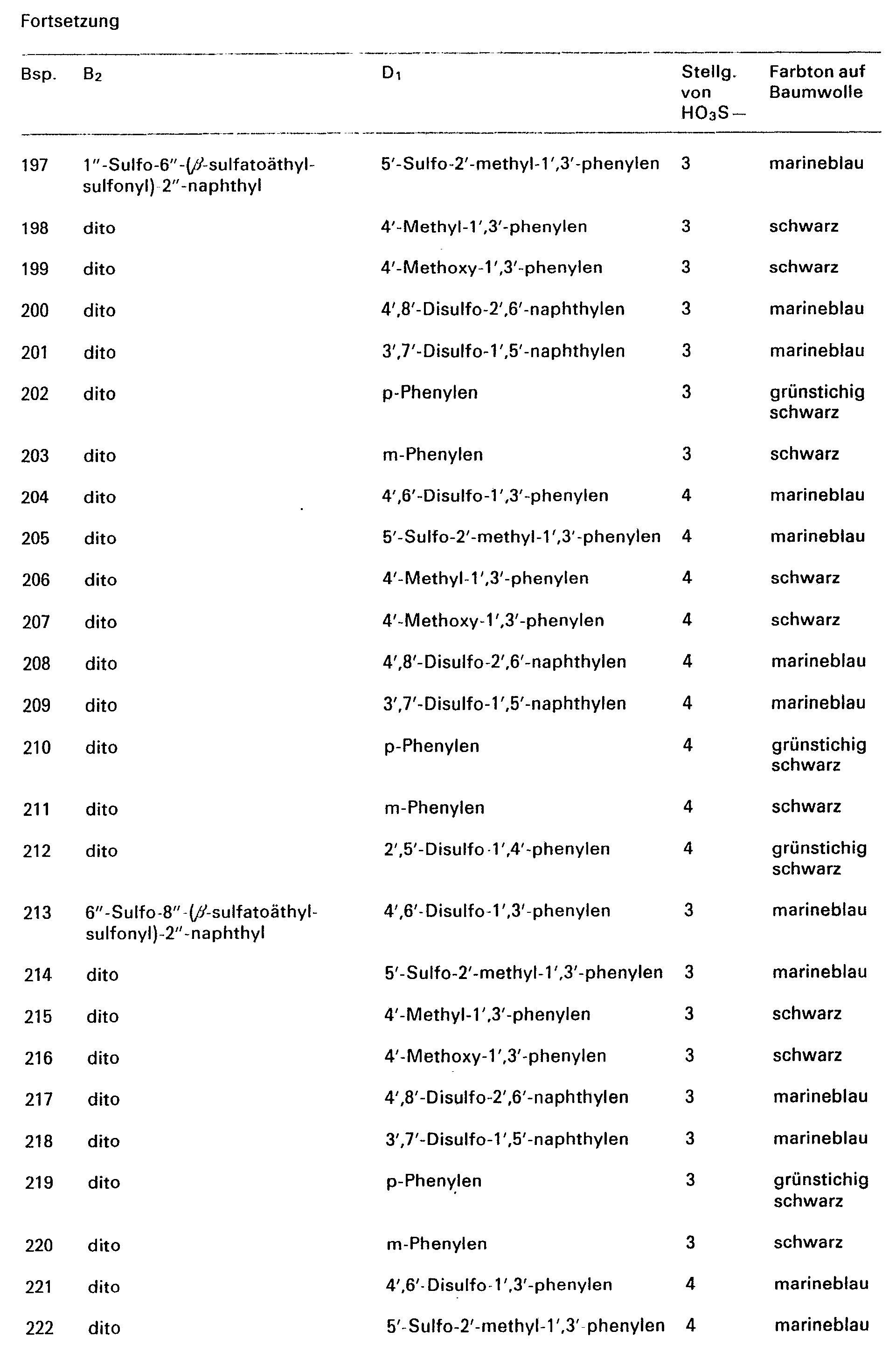

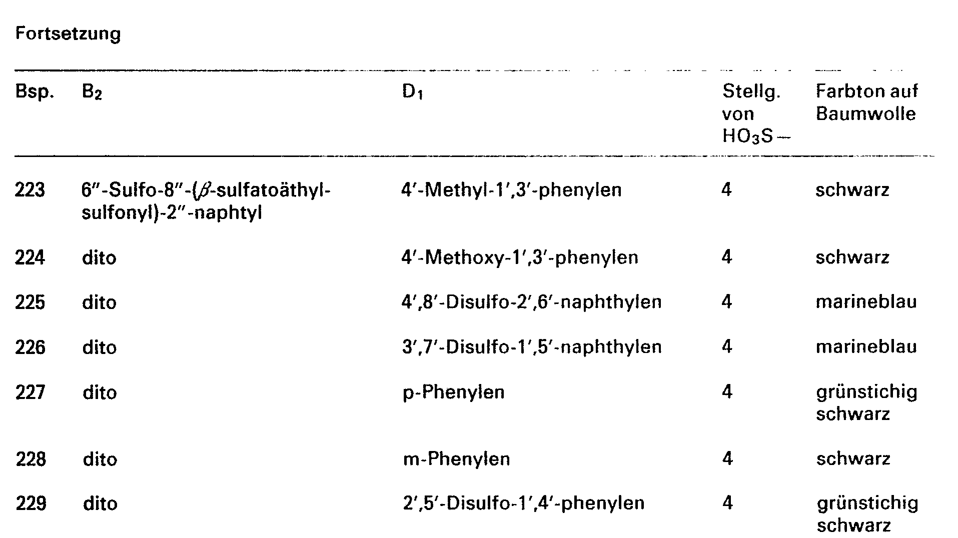

### Beispiel 230

Zu einer neutralen Lösung von 28,1 Teilen Anilin-4-,8-sulfatoäthylsulfon in 100 Teilen Wasser werden bei 0-3° C innerhalb von 15 Minuten 13,7 Gewichtsteile Cyanurfluorid zugetropft, wobei zur Einhaltung des pH-Wertes bei etwa 6,0 wäßriges Natriumhydroxid zugegeben wird. Anschließend wird zu dem erhaltenen Reaktionsgemisch eine neutrale Lösung von 26,8 Gewichtsteilen 1,4-Diamino-benzol-2,5-disulfonsäure in 200 Teilen Wasser zugefügt und das so gewonnene Gemisch bei 18-20"C und einem pH-Wert von ca. 6,0 bis 7,0 einige Stunden gerührt, wobei der pH-Wert durch portionsweise Zugabe von wäßrigem Natriumhydroxid im angegebenen Bereich gehalten wird. Das gebildete Reaktionsprodukt liegt dann in einer klaren Lösung vor.

Zu dieser Lösung gibt man 250 Teile Eis und 60 Volumenteile einer 31 %igen wäßrigen Salzsäure und diazotiert sodann bei einer Temperatur von 0 bis 5° C durch tropfenweise Zugabe von 20 Volumenteilen einer wäßrigen 5 n-Natriumnitritlösung. Die erhaltene Diazoniumsalzlösung wird bei einer Temperatur zwischen 0 und 10°C und bei einem pH-Wert von 2,5 bis 3,0 in eine Lösung von 31,9 Teilen 1-Amino-8-naphthol-3,6-disulfonsäure in 1500 Teilen Wasser eingetropft, wobei der pH-Wert durch portionsweises Eintragen von 63 Teilen kristallisiertem Natriumacetat im angegebenen Bereich gehalten wird. Nach beendeter Kupplung wird die gebildete Monoazoverbindung mittels Kaliumchlorid ausgefällt und abgesaugt.

Der feuchte Filterkuchen der Monoazoverbindung wird in 500 Teilen Wasser gelöst und diese Lösung mit einer Diazoniumsalzlösung versetzt, die wie nachfolgend angegeben hergestellt wird:
Zu einer neutralen Lösung von 34,1 Teilen 2,5-Dimethoxyanilin-4-β sulfatoäthyl-sulfon in einem Gemisch von 140 Teilen Wasser und 60 Teilen Eis werden bei 0-2"C innerhalb von 15 Minuten 13,7 Teile Cyanurfluorid zugetropft, wobei zur Einhaltung des pH-Wertes bei ca. 6,0 wäßriges Natriumhydroxid zugegeben wird. Anschließend wird zu dem erhaltenen Reaktionsgemisch eine neutrale Lösung von 18,8 Gewichtsteilen 1,4-Diaminobenzol-2-sulfonsäure in 200 Teilen Wasser zugefügt und das so gewonnene Gemisch bei 18-20°C und einem pH-Wert von 6,2 bis 6,8 einige Stunden gerührt, wobei der pH-Wert durch portionsweise Zugabe von wäßrigem Natriumhydroxid im angegebenen Bereich gehalten wird. Die erhaltene Lösung des sekundären Kondensationsproduktes wird anschließend mit 40 Volumenteilen einer 31%igen wäßrigen Salzsäure versetzt, auf 0 bis 5"C gekühlt und mittels 20 Volumenteilen einer wäßrigen 5 n-Natriumnitritlösung diazotiert. Diese Diazoniumsalzlösung wird mit Natriumbicarbonat auf einen pH-Wert von 5,5 bis 5,8 gestellt und, wie oben erwähnt, zu der Lösung der Monoazoverbindung gegeben.

Diese zweite Kupplungsreaktion führt man bei einem pH-Wert von 5,5 bis 6,5 und bei einer Temperatur von 23 bis 25°C durch. Die gebildete Disazoverbindung wird mittels Kaliumchlorid ausgefällt, abgesaugt, getrocknet und gemahlen. Man erhält ein schwarzes, elektrolythaltiges, vorwiegend Kaliumchlorid enthaltendes Pulver, welches zu etwa 48% das Alkalimetallsalz, vorwiegend Kaliumsalz, der Verbindung der Formel 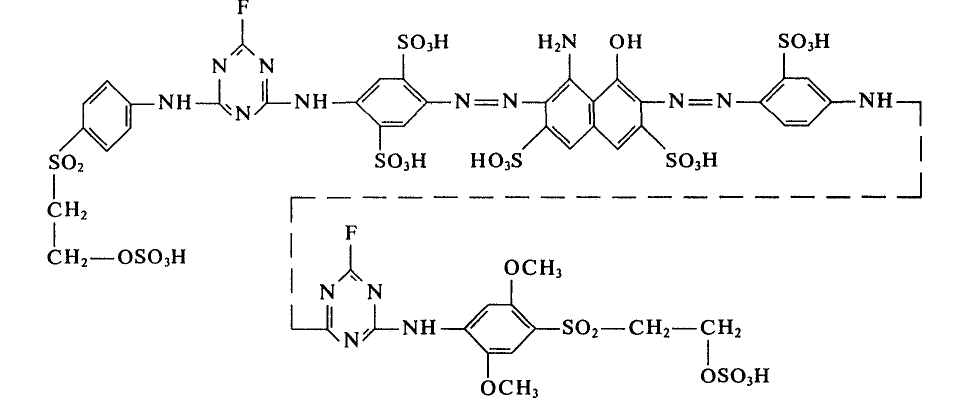 enthält. Diese Verbindung besitzt sehr gute Farbstoffeigenschaften und liefert nach den für faserreaktive Farbstoffe üblichen Applikations- und Fixierverfahren auf Cellulosefasermaterialien Färbungen und Drucke mit grünstichig schwarzer Nuance von sehr guten Gebrauchs- und Fabrikationsechtheiten, wie insbesondere die sehr gute Waschechtheit, Schweiß-, Wasser-, Meerwasser-, Säure- und Alkaliechtheit, weiterhin sehr gute Reib- und Plissierechtheiten.

### Beispiele 231 bis 237

Verfährt man in erfindungsgemäßer Verfahrensweise zur Herstellung der erfindungsgemäßen Verbindungen der allgemeinen Formel (1) analog den Angaben des Beispiels 230, setzt jedoch anstelle der dort angegebenen Reaktionskomponenten die aus den nachfolgenden Tabellenbeispielen in Verbindung mit der allgemeinen Formel (C) ersichtlichen Ausgangskomponenten (Anilinderivate mit der jJ-Sulfatoäthylsulfonylgruppe, Cyanurfluorid und einer Diaminobenzol-mono- oder -disulfonsäure für die beiden sekundären Kondensationsprodukte, die als Diazokomponente dienen, sowie die 1-Amino-8-naphthol-3,6- oder -4,6-disulfonsäure als Kupplungskomponente) ein, so erhält man die in diesen Tabellenbeispielen unter Bezugnahme auf die allgemeine Formel (C) genannten erfindungsgemäßen Disazoverbindungen, die sehr gute Farbstoffeigenschaften besitzen und nach den in der Technik üblichen Applikations- und Fixiermethoden für faserreaktive Farbstoffe Färbungen und Drucke mit guten Echtheiten in den in den Tabellenbeispielen angegebenen Farbtönen liefern.

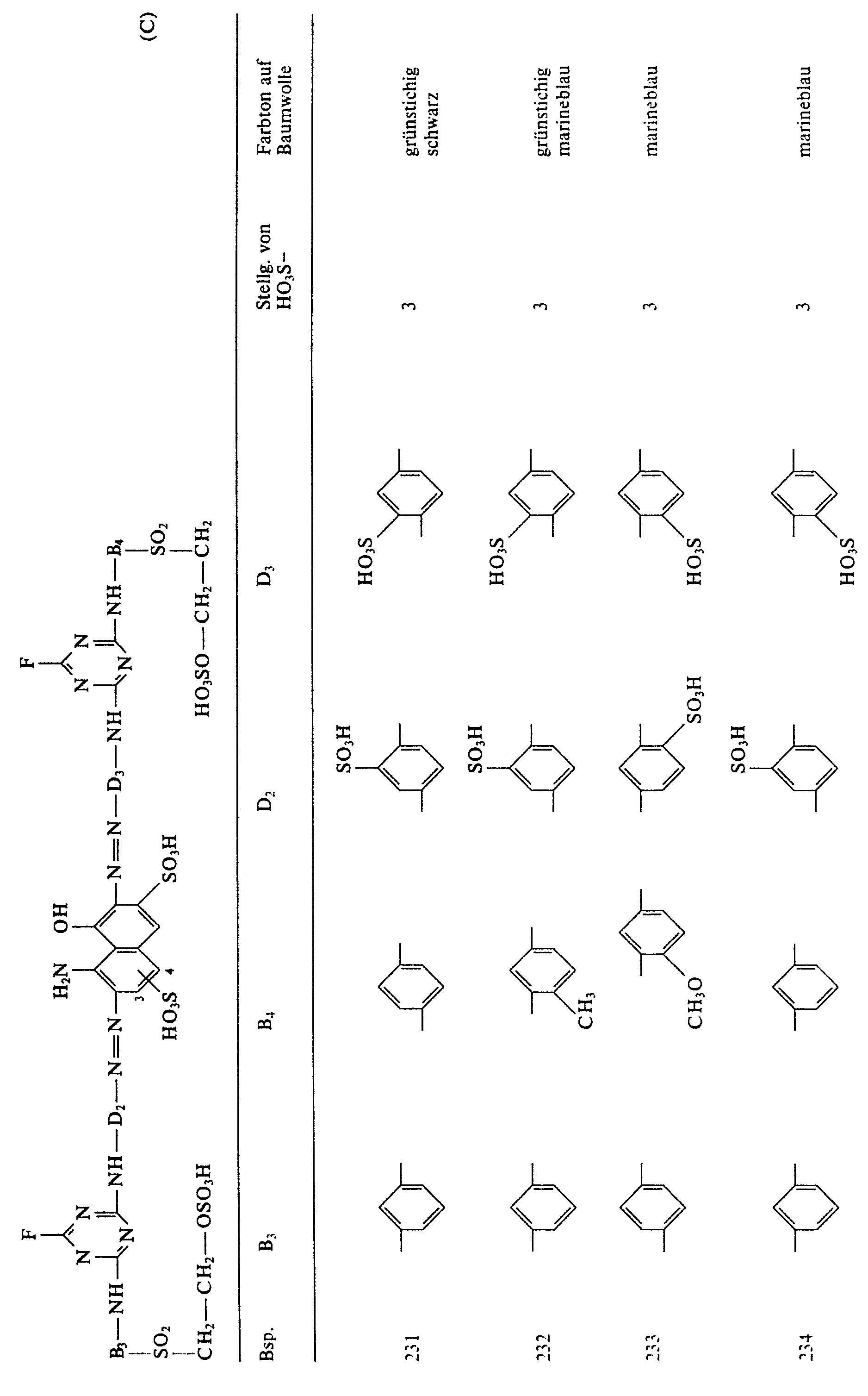

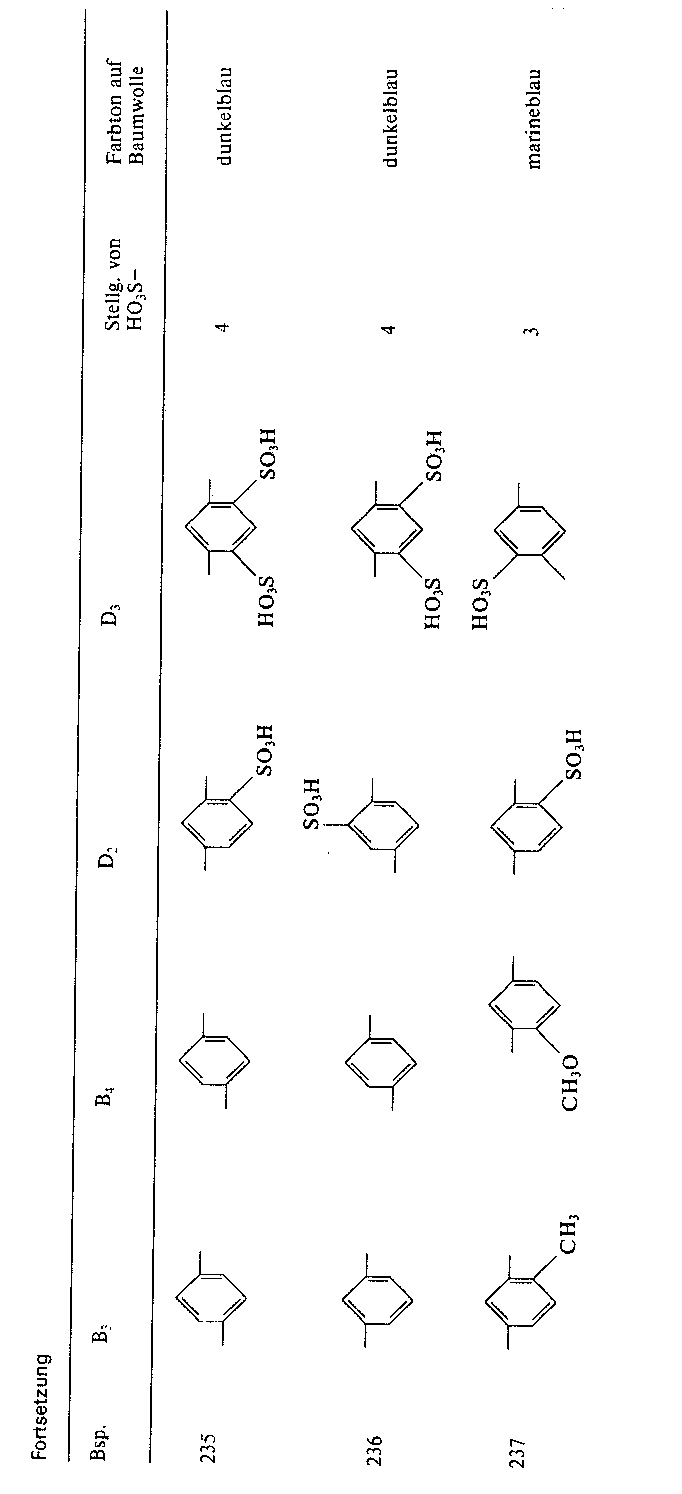

### Beispiel 238

Man verfährt in der im Beispiel 1 angegebenen Verfahrensweise, ersetzt jedoch das Anilin-4-β-sulfatoäthylsulfon jeweils durch die äquivalente Menge (29,7 Teile) Anilin-4-β-thiosulfatoäthylsulfon. Es wird ein elektrolythaltiges Farbstoffpulver erhalten, welches das Alkalimetallsalz der Verbindung der Formel 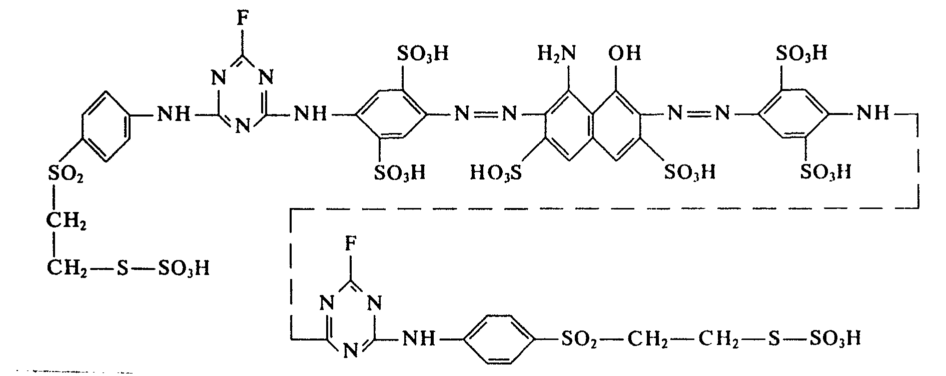 enthält. Diese Disazoverbindung stellt ebenfalls einen sehr guten Farbstoff dar, der im Vergleich zu dem Farbstoff des Beispiels 1 ähnlich gute coloristische Eigenschaften und Echtheiten aufweist. Er läßt sich auf natürlichen und synthetischen Polyamidfasermaterialien, insbesondere jedoch auf natürlichen und regenerierten Cellulosefasermaterialien, wie vorzugsweise Baumwolle, nach den insbesondere fürfaserreaktive Farbstoffe üblichen Applikations- und Fixiermethoden anwenden.

### Beispiel 239

Man stellt gemäß den Angaben des Beispiels 1 die Monoazoverbindung her und löst sie in 500 Teilen Wasser. Die zweite Kupplungsreaktion wird mit einer anderen Diazoniumverbindung durchgeführt, die wie folgt hergestellt wird: Zu einer Lösung von 17,3 Teilen Anitin 2 sulfonsäure in 100 Teilen Wasser und 50 Teilen einer 2 n-Natriumhydroxidlösung werden bei 0 C innerhalb von 10 bis 20 Minuten 13,7 Teile Cyanurfluorid zugetropft, wobei zur Einhaltung des pH-Wertes bei ca. 6,0 wäßriges Natriumhy droxid zugegeben wird. Anschließend fügt man eine neutrale Lösung von 18,8 Teilen 1,4-Diaminobenzol-2-sulfonsäure in 200 Teilen Wasser hinzu und läßt die zweite Kondensationsreaktion bei einem pH-Wert von 6,0 bis 7,0 und einer Temperatur von 18 bis 20"C unter mehrstündigem Rühren ablaufen. Nach beendeter Kondensation wird die Lösung geklärt, mit 35 Volumenteilen einer 31%igen wäßrigen Salzsäure angesäuert, mit 500 Teilen Eis versetzt und durch langsame Zugabe von 20 Volumenteilen einer wäßrigen 5 n-Natriumnitritlösung diazotiert. Wie üblich, wird danach überschüssige salpetrige Säure mit etwas Amidosulfonsäure zerstört. Die Diazoniumsalzsuspension wird sodann mit 17,8 Teilen Natriumbicarbonat auf einen pH-Wert von 5,5 bis 6,5 eingestellt und, wie oben erwähnt, mit der Lösung der Monoazoverbindung bei einem pH-Wert von 5,5 bis 6,5 gekuppelt. Die gebildete Disazoverbindung wird mit Kaliumchlorid ausgesalzen, abgesaugt und getrocknet. Es wird ein schwarzes, elektrolythaltiges, vorwiegend Kaliumchlorid enthaltendes, Farbstoffpulver erhalten, das zu etwa 40 bis 45% das Alkalimetallsalz, vorwiegend Kaliumsalz der Verbindung der Formel 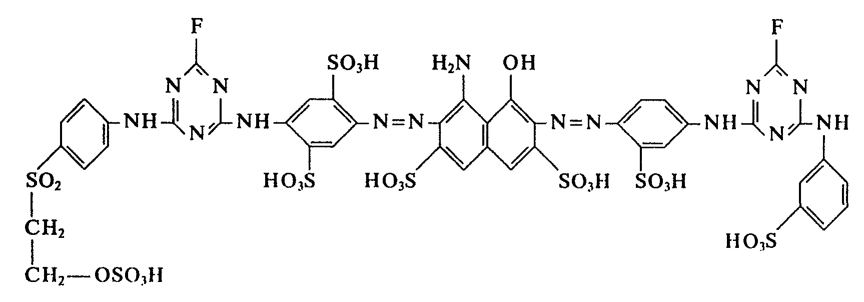 enthält. Diese Verbindung zeigt sehr gute Farbstoffeigenschaften und liefert nach dan für fasorreakti ve Farbstoffe üblichen Applikations- und Fixiermethoden auf Cellulosefasermaterialien, wie Baumwolle, grünstichig schwarze Färbungen und Drucke mit sehr guten Gebrauchs- und Fabrikationsechtheiten, wie beispielsweise die sehr gute Waschechtheit, Schweiß-, Reib-, Säure- und Alkaliechtheit.

### Beispiele 240 bis 255

Verfährt man in erfindungsgemäßer Verfahrensweise zur Herstellung der erfindungsgemäßen Verbindungen der allgemeinen Formel (1) analog den Angaben des Beispiels 239, setzt jedoch anstelle der dort angegebenen Reaktionskomponenten die aus den nachfolgenden Tabellenbeispielen in Verbindung mit den allgemeinen Formeln (D) und (E) ersichtlichen Ausgangskomponenten (Anilinderivate, die eine d-Sulfatoäthylsulfonylgruppe besitzen oder von dieser frei sind, Cyanurfluorid und eine Diaminobenzol-Verbindung für die beiden sekundären Kondensationsprodukte, die als Diazokomponente dienen, sowie die 1-Amino-8-naphthol-3,6- oder -4,6-disulfonsäure als Kupplungskomponenten) ein, so erhält man die in diesen Tabellenbeispielen unter Bezugnahme auf die allgemeinen Formeln (D) und (E) genannten erfindungsgemäßen Disazoverbindungen, die sehr gute Farbstoffeigenschaften besitzen und nach den in der Technik üblichen Applikations- und Fixiermethoden für faserreaktive Farbstoffe Färbungen und Drucke mit guten Echtheiten in den in den Tabellenbeispielen angegebenen Farbtönen liefern.

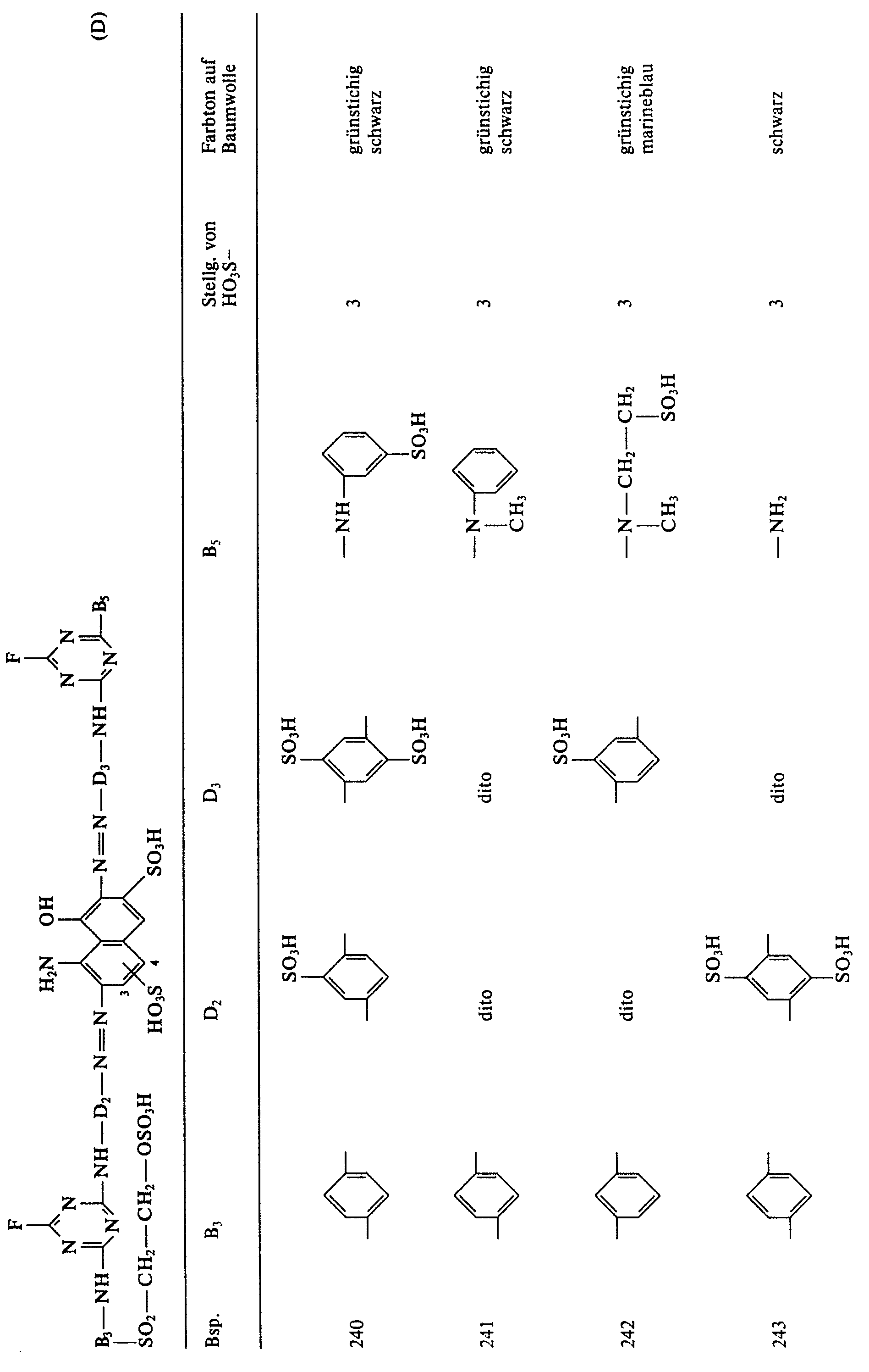

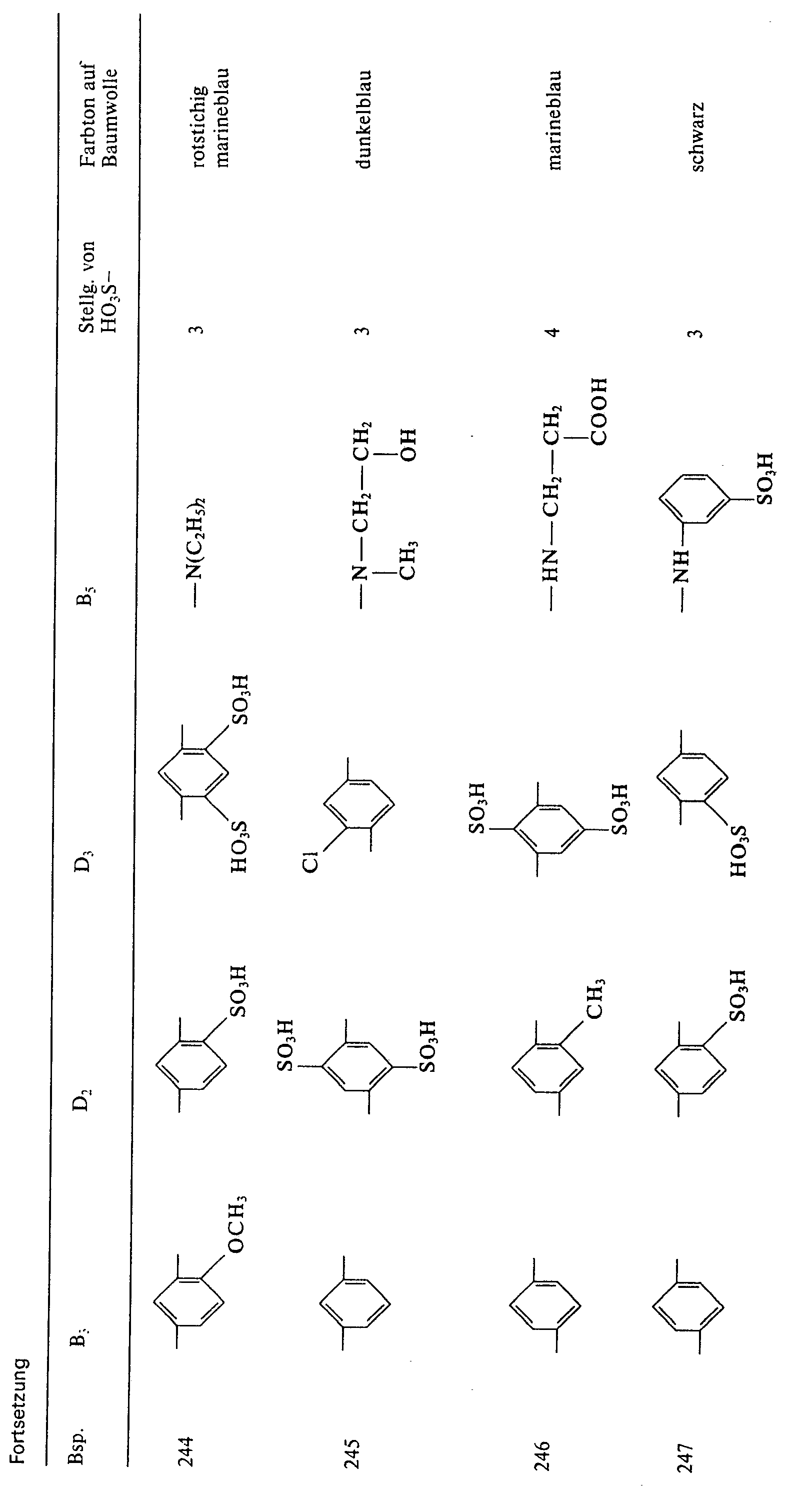

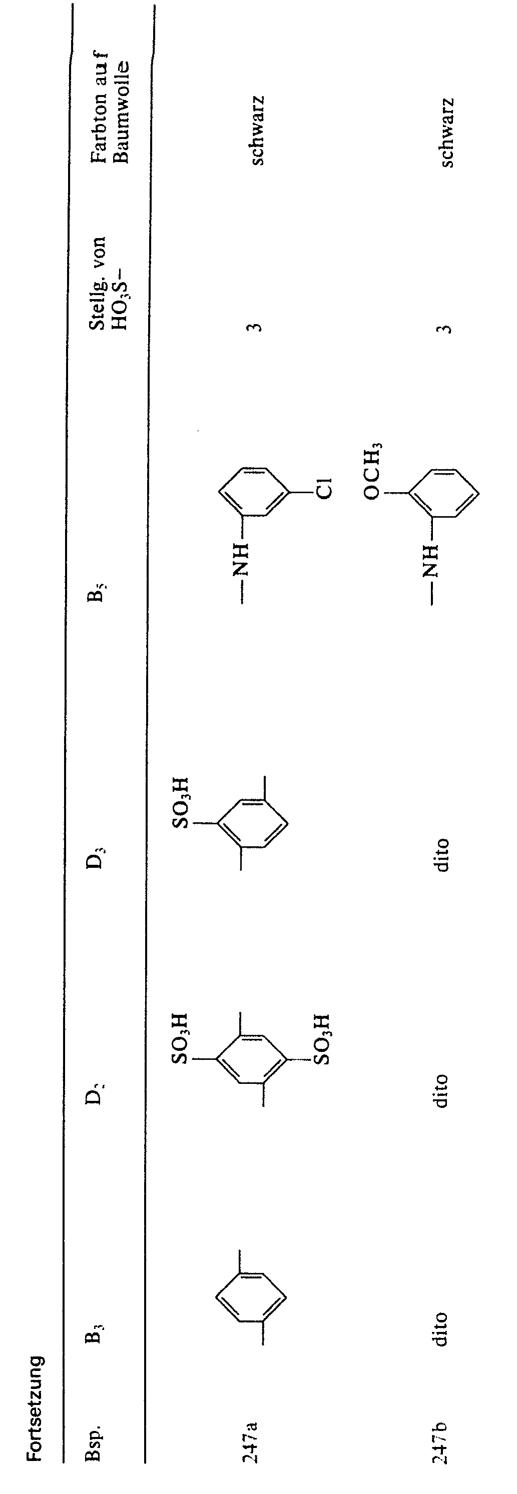

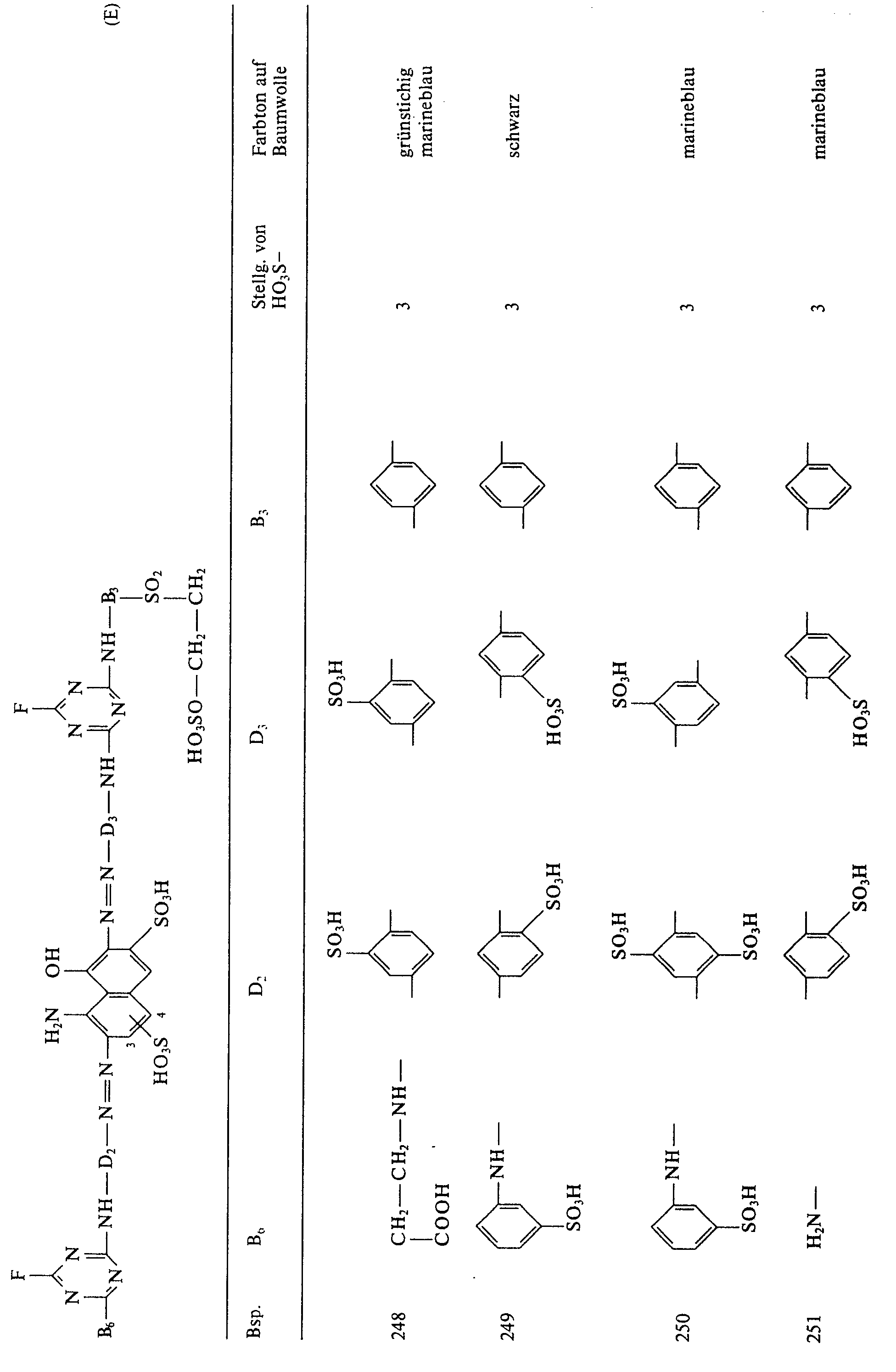

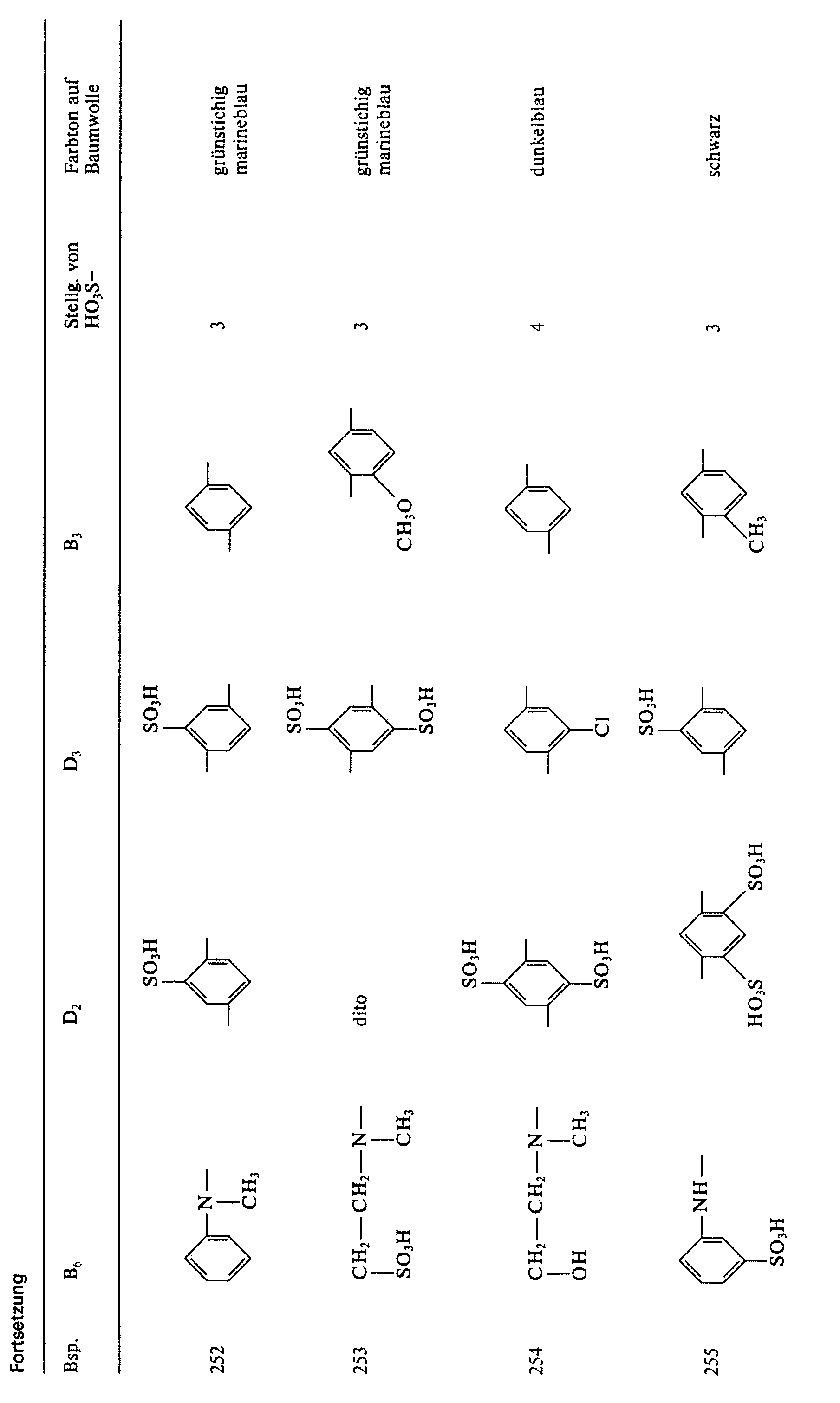

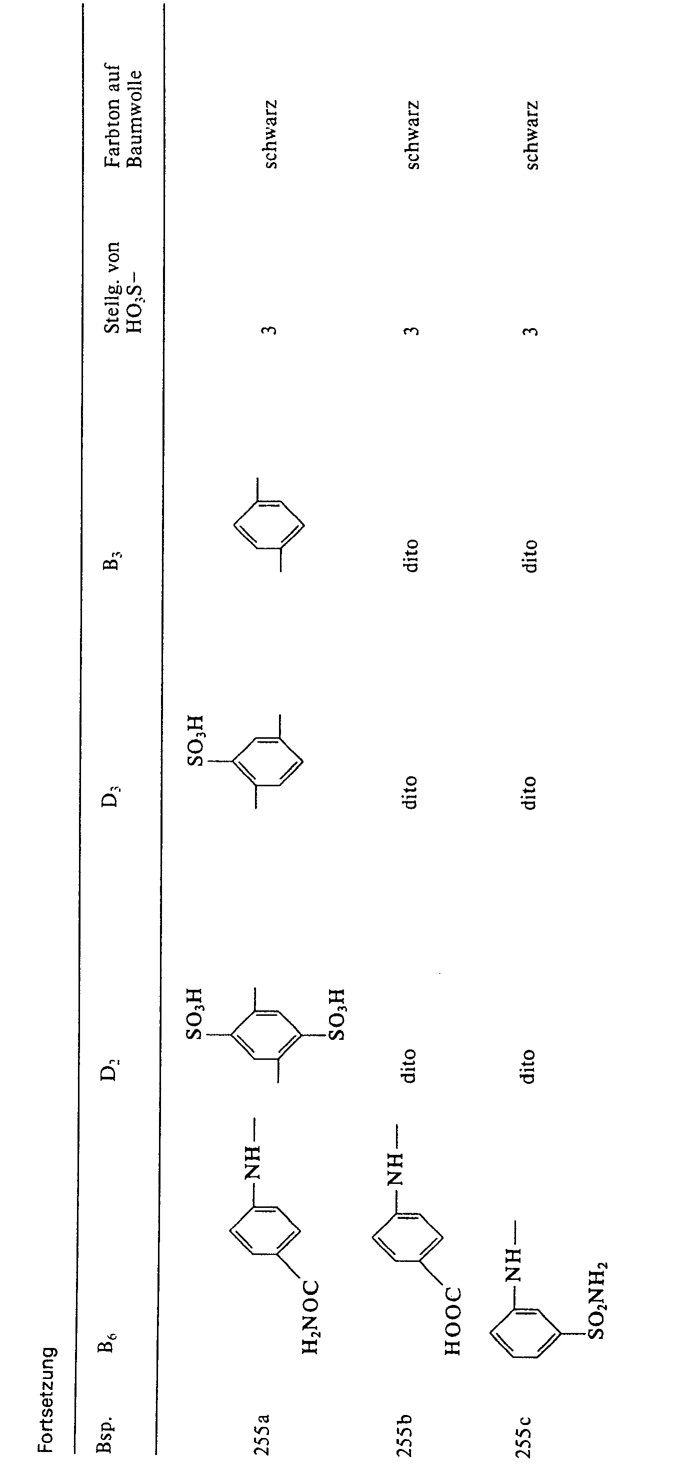

### Beispiel 256

Gemäß den Angaben des Beispiels 1 wird die Monoazoverbindung hergestellt und in 500 Teilen Wasser gelöst. Die zweite Kupplungsreaktion erfolgt mit einem Diazoniumsalz, das wie nachstehend beschrieben hergestellt wird: 28,1 Teile Anilin-4-;J-sulfatoäthylsulfon werden in einem Gemisch aus 100 Teilen Wasser und 50 Teilen Eis suspendiert und durch Zugabe von 7,3 Teilen Natriumcarbonat neutral gelöst. Sodann fügt man 20,3 Volumenteile einer wäßrigen 5 n-Natriumnitritlösung hinzu und läßt dieses Gemisch auf eine Mischung aus 26 Volumenteilen einer 310/oigen wäßrigen Salzsäure und 150 Teilen Eis laufen. Die erhaltene Suspension wird noch eine Stunde weitergerührt, sodann überschüssige salpetrige Säure mit Amidosulfonsäure zerstört und das Reaktionsprodukt mit 7 Teilen Natriumbicarbonat auf einen pH-Wert von 5,7 bis 6,2 gestellt.

Die Lösung der Monoazoverbindung wird, wie oben erwähnt, zu der so hergestellten Diazoniumsalzsuspension gegeben, wobei der pH-Wert durch portionsweise Zugabe von 16,3 Teilen Natriumbicarbonat auf 5,7 bis 6,2 gehalten wird. Nach mehrstündigem Rühren ist die Kupplung beendet und die gebildete Disazoverbindung wird mit Kaliumchlorid ausgesalzen, abgesaugt und getrocknet.

Es wird ein schwarzes, elektrolythaltiges Pulver erhalten, das zu etwa 45% das Alkalimetallsalz, vorwiegend Kaliumsalz, der Verbindung der Formel 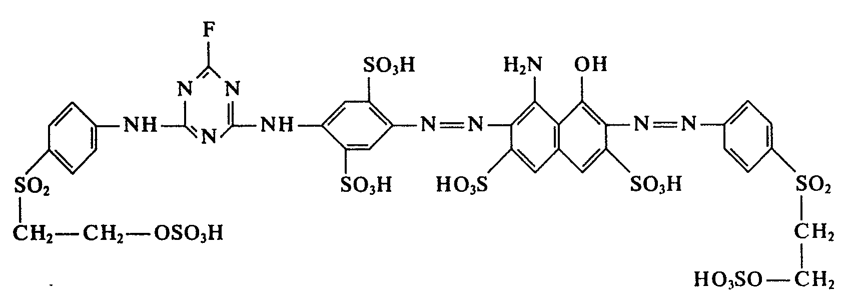 enthält. Diese zeigt sehr gute Farbstoffeigenschaften und liefert nach den für faserreaktive Farbstoffe üblichen Applikations- und Fixiermethoden auf Cellulosefasermaterialien kräftige schwarze Färbungen von sehr guten Gebrauchs- und Fabrikationsechtheiten, wie insbesondere die sehr gute Wasch-, Meerwasser- und Schweißechtheit. Daneben weist der Farbstoff einen sehr hohen Fixiergrad und eine gute Auswaschbarkeit der nicht fixierten Anteile auf.

### Beispiele 257 bis 265

Man verfährt in erfindungsgemäßer Weise analog der in Beispiel 256 beschriebenen Verfahrensweise zur Herstellung einer erfindungsgemäßen Disazoverbindung, setzt jedoch anstelle der dort angegebenen Reaktionskomponenten die aus den nachfolgenden Tabellenbeispielen in Verbindung mit der allgemeinen Formel (F) ersichtlichen Reaktionskomponenten (Anilinverbindung mit einer β-Sulfatoäthylsulfonylgruppe, Cyanurfluorid und eine Diaminobenzolverbindung zur Herstellung des als Diazokomponente dienenden Kondensationsproduktes, 1-Amino-8-naphthol-3,6- oder -4,6-disulfonsäure und eine Anilinverbindung mit einer β-Sulfatoäthylsulfonylgruppe als zweite Diazokomponente) ein, so erhält man die in den Tabellenbeispielen unter Bezugnahme auf die Formel (F) angegebenen erfindungsgemäßen Disazoverbindungen, die ebenfalls sehr gute Farbstoffeigenschaften besitzen und nach den für faserreaktive Farbstoffe üblichen Applikations- und Fixiermethoden auf bevorzugt Cellulosefasermaterialien farbkräftige Färbungen und Drucke mit den in den Beispielen angegebenen Nuancen liefern. Auch diese Färbungen und Drucke zeichnen sich durch gute Echtheiten aus.

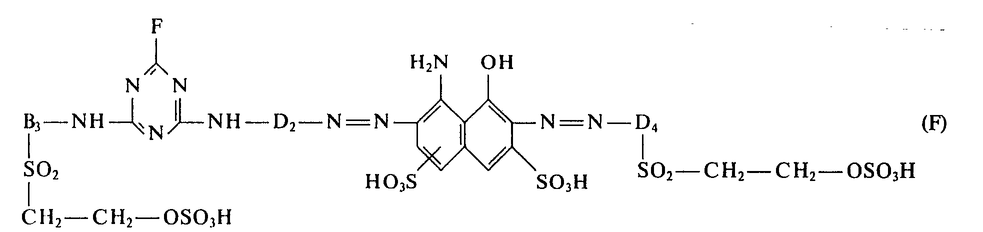

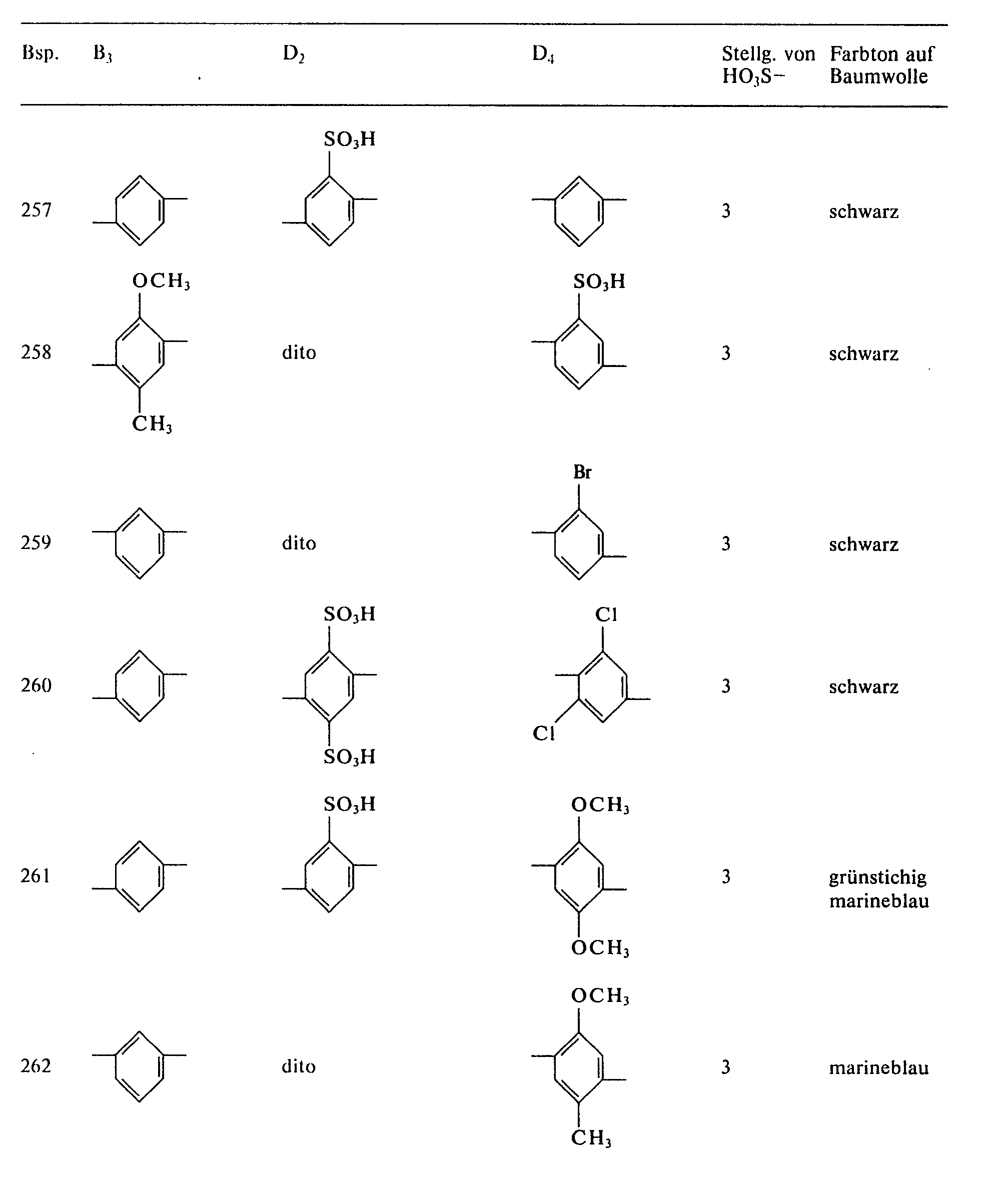

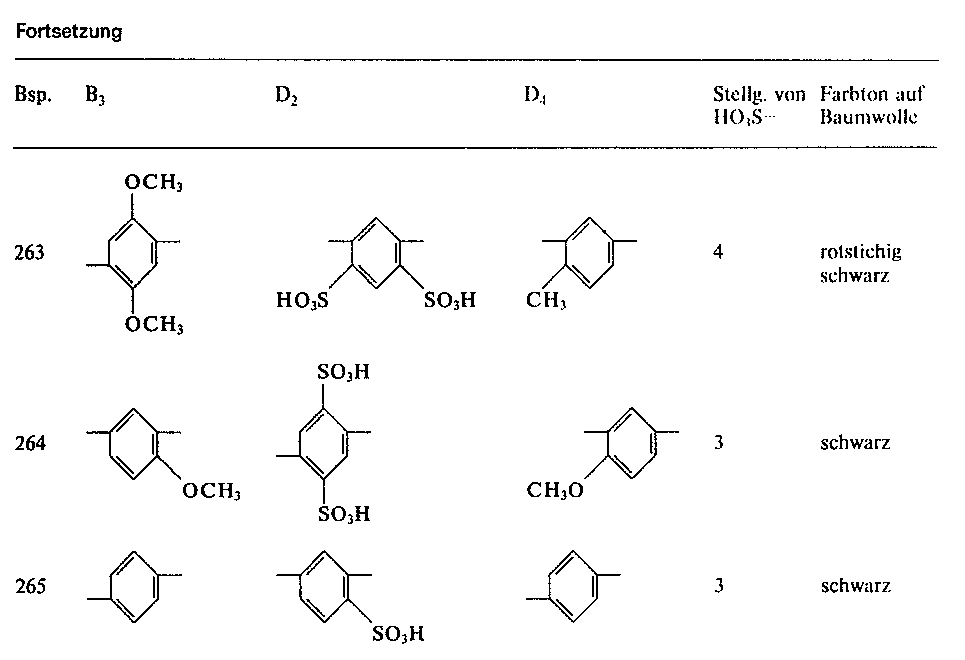

### Beispiel 266

Ein Gemisch aus einer neutralen Lösung von 28,1 Teilen Anilin-4-β-sulfatoäthylsulfon in 150 Teilen Wasser und 20,3 Volumenteilen einer wäßrigen 5 n-Natriumnitritlösung läßt man bei 0 bis 5°C unter Rühren in ein Gemisch aus 26 Volumenteilen einer 31%igen wäßrigen Salzsäure und 150 Teilen Eis einlaufen. Die entstehende Suspension wird noch eine Stunde gerührt und überschüssige salpetrige Säure mit Amidosulfonsäure zerstört. Sodann wird zur Kupplungsreaktion eine Lösung von 31,9 Teilen 1-Amino-8-naphthol-3,6 disulfonsäure in 500 Teilen Wasser, die mit Salzsäure auf einen pH-Wert von 4 gestellt ist, versetzt. Der pH-Wert der Kupplung wird mit Natriumacetat bei 3 bis 3,5 gehalten. Nachdem die erste Kupplungsreaktion beendet ist, wird die gebildete Monoazoverbindung mit einer Diazoniumsalzlösung in der zweiten Kupplungsreaktion umgesetzt. Diese Diazoniumsalzlösung wird wie folgt hergestellt: Zu einer neutralen Lösung von 28,1 Teilen Anilin-4-,9-sulfatoäthylsulfon in 100 Teilen Wasser läßt man 13,7 Teile Cyanurfluorid bei 0°C innerhalb von 10-15 Minuten zutropfen, wobei zur Einhaltung des pH-Wertes von ca. 6,0 wäßriges Natriumhydroxid zugegeben wird.

Die so hergestellte Suspension des primären Kondensationsproduktes wird anschließend mit einer neutralen Lösung von 26,8 Teilen 1,4-Diaminobenzol-2,5-disulfonsäure in 200 Teilen Wasser versetzt und bei 18 bis 20" C und einem pH-Wert von 6,0 bis 7,0 gerührt. Zu der so erhaltenen klaren Lösung des sekundären Kondensationsproduktes werden 250 Teile Eis und danach 60 Teile einer wäßrigen 31%igen Salzsäure gegeben, und das Kondensationsprodukt wird bei 0 bis 5°C durch langsame Zugabe von 20 Volumenteilen einer wäßrigen 5 n-Natriumnitritlösung diazotiert.

Diese Diazoniumsalzlösung wird mit Natriumbicarbonat auf einen pH-Wert von 5,5 bis 6 gestellt und, wie oben erwähnt, mit der Monoazoverbindung gekuppelt, wobei die Kupplungsreaktion bei einem pH-Wert von 5,8 bis 6,2 durchgeführt wird. Nach mehrstündigem Rühren wird die gebildete Disazoverbindung mit Kaliumchlorid ausgefällt, abgesaugt und getrocknet.

Es wird ein schwarzes, elektrolythaltiges Pulver erhalten, welches das Alkalimetallsalz, vorzugsweise Kaliumsalz, der Verbindung der Formel 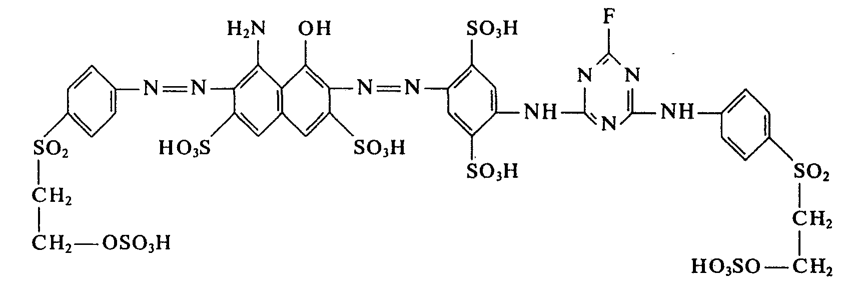 zu etwa 50% enthält. Diese Disazoverbindung besitzt sehr gute Farbstoffeigenschaften und färbt nach den für Reaktivfarbstoffe üblichen Färbe- und Druckverfahren Cellulosefasermaterialien in schwarzen Tönen mit sehr guten Gebrauchs- und Fabrikationsechtheiten.

### Beispiele 267 bis 273

Verfährt man in erfindungsgemäßer Weise zur Herstellung von erfindungsgemäßen Disazoverbindungen, so beispielsweise analog einer der oben angegebenen Verfahrensweisen der Ausführungsbeispiele, vorzugsweise analog dem Beispiel 266, und setzt hierzu entsprechende Reaktionskomponenten (als erste Diazokomponente ein Anilin mit einer β-Sulfatoäthylsulfonylverbindung, als zweite Diazokomponente ein Reaktionsprodukt aus einer Anilinverbindung mit einer β-Sulfatoäthylsulfonyl- gruppe, Cyanurfluorid und einer Diaminobenzol-Verbindung sowie als Kupplungskomponente 1-Amino-8-naphthol-3,6- oder -4,6-disulfonsäure) ein, wie sie aus den nachfolgenden Tabellenbeispielen in Verbindung mit der allgemeinen Formel (G) ersichtlich sind, so erhält man die in den nachfolgenden Tabellenbeispielen mit den dort angegebenen Formelresten der Formel (G) gekennzeichneten wertvollen Disazoverbindungen entsprechend der allgemeinen Formel (1), die ebenfalls sehr gute Farbstoffeigenschaften besitzen und nach in der Technik üblichen Applikations- und Fixiermethoden für faserreaktive Farbstoffe auf Cellulosefasermaterialien kräftige, echte Färbungen und Drucke mit den in den Beispielen angegebenen Farbtönen liefern.

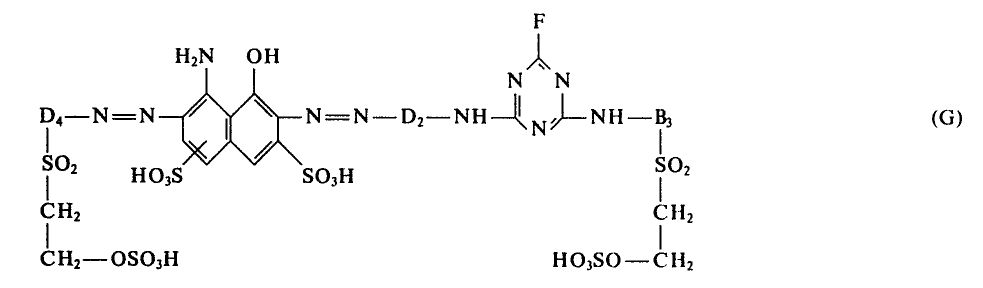

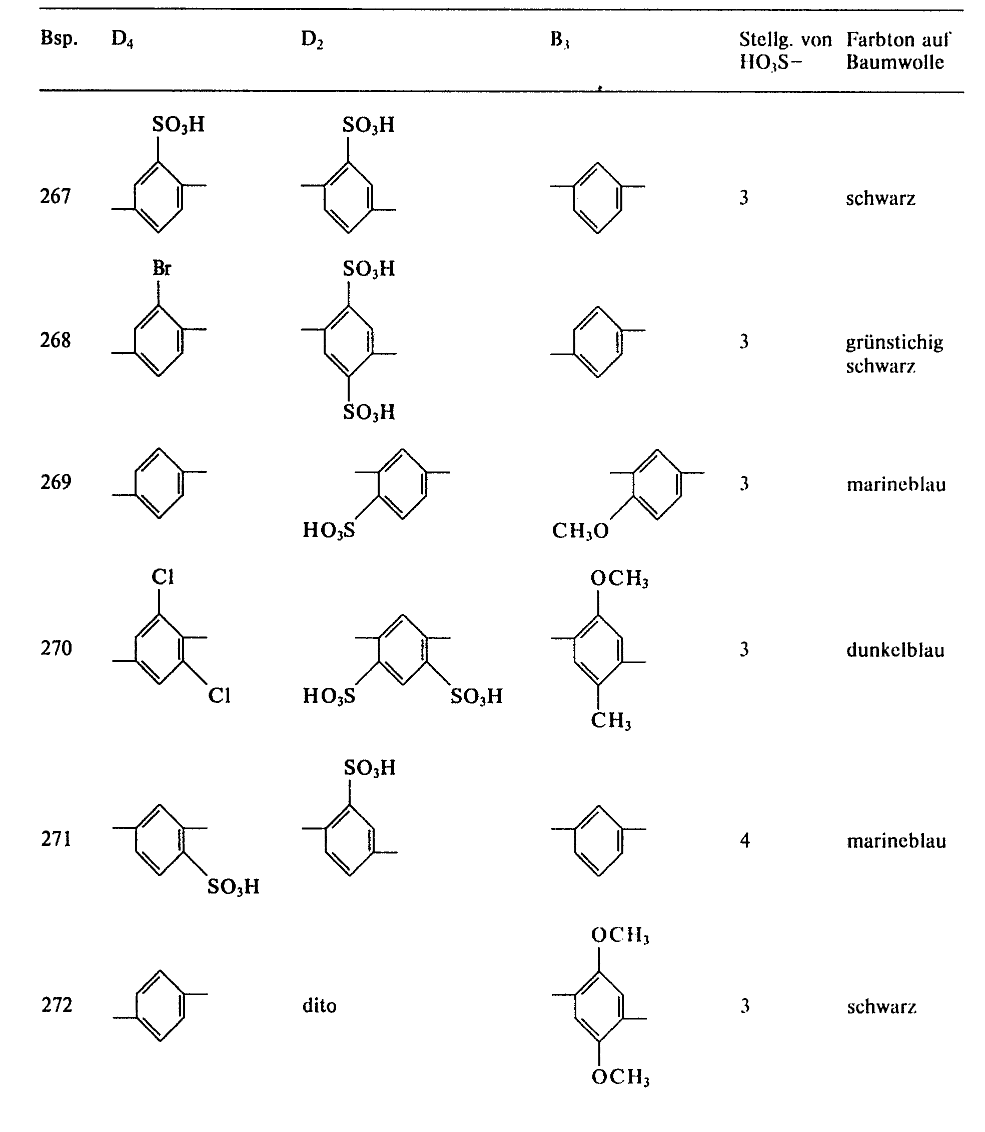

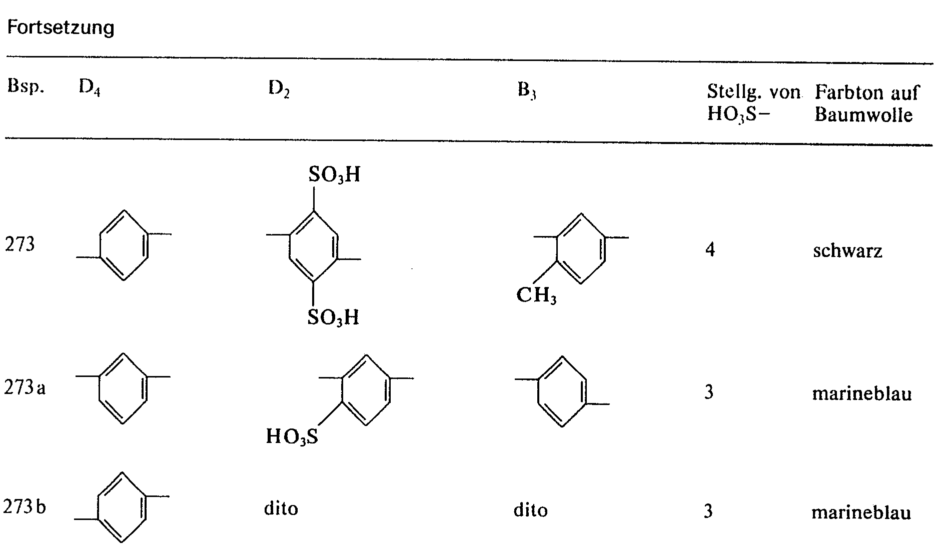

### Beispiel 274

Man verfährt zur Herstellung einer erfindungsgemäßen Disazoverbindung gemäß den Angaben des Beispiels 266, setzt jedoch anstelle der Anilin-4-β-sulfatoäthylsulfon-Verbindung 26,5 Teile Anilin-4-β phosphatoäthylsulfon ein. Man erhält das Alkalimetallsalz der Verbindung der Formel 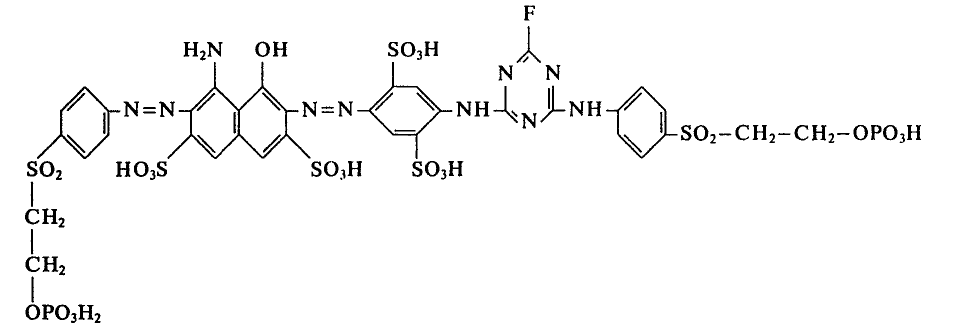 das ähnlich gute Farbstoffeigenschaften wie der Disazofarbstoff des Beispiels 266 besitzt.

## Patentansprüche

1. Wasserlösliche Disazoverbindungen der allgemeinen Formel (1) 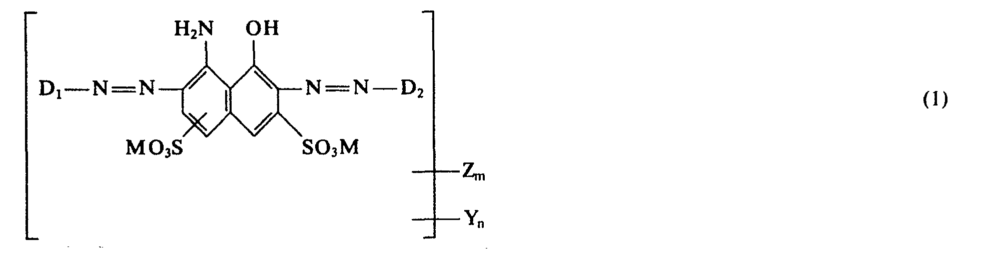 in welcher bedeuten:
D₁ und Dz stellt jedes den Phenylrest oder einen Naphthylrest dar, die beide durch eine Gruppe des nachstehend definierten Formelrestes Y oder Formelrestes Z substituiert sind und zusätzlich durch einen oder zwei Substituenten aus der Gruppe Sulfo, Chlor, Brom, niederes Alkyl und niederes Alkoxy substituiert sein können,
wobei Di und Dz zueinander gleiche oder voneinander verschiedene Bedeutungen besitzen können;

Z ist eine Gruppe der Formel (2a), (2b), (2c), (2d), (2e) oder (2f) 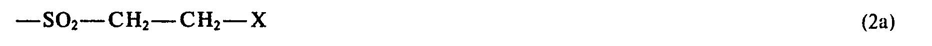 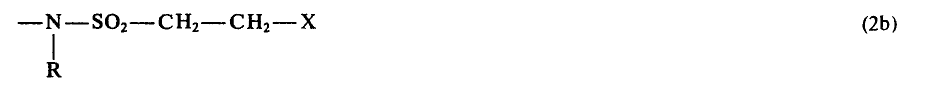 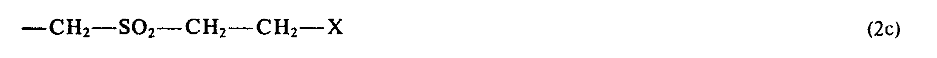 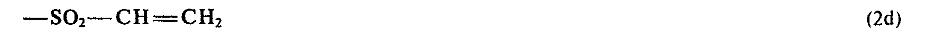 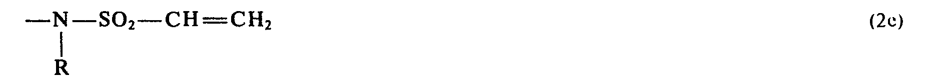 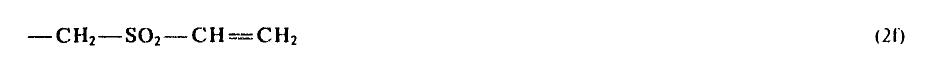 in welchen
R eine Alkylgruppe von 1 bis 4 C-Atomen und
X ein Chloratom, die Acetyloxygruppe, die Thiosulfatogruppe, die Phosphatogruppe oder die Sulfatogruppe bedeutet;
Y ist ein Rest der Formel (3) 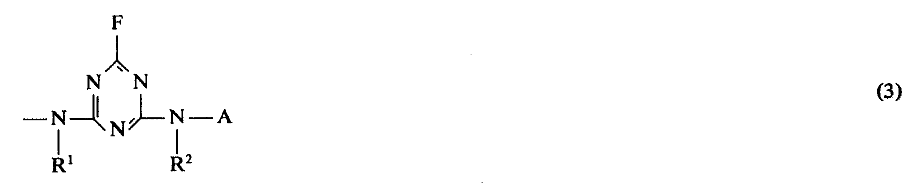 in welcher
R^{l} ein Wasserstoffatom oder eine Alkylgruppe von 1 bis 4 C-Atomen und
R² ein Wasserstoffatom oder eine Alkylgruppe von 1 bis 4 C-Atomen ist,
wobei R¹ und R² zueinander gleiche oder voneinander verschiedene Bedeutungen besitzen können, sowie
A ein Wasserstoffatom oder eine Alkylgruppe von 1 bis 6 C-Atomen ist, die substituiert sein kann, oder der Phenylrest ist, der durch Substituenten aus der Gruppe Methyl, Äthyl, Methoxy, Äthoxy, Chlor, Brom, Carboxy, Sulfo, Carbamoyl und Sulfamoyl substituiert sein kann, oder ein Rest der Formel (4) 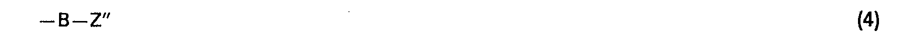 ist, worin
B der Phenylenrest oder ein Napthylenrest ist, die beide durch einen oder zwei Substituenten substituiert sein können, die aus der Menge von einer Sulfogruppe, einem Chloratom, . einer oder zwei Methyl- oder Äthylgruppen und einer oder zwei Methoxy- oder Äthoxygruppen ausgewählt sind, und
Z" eine der für Z genannten Bedeutungen hat;
n ist die Zahl 1 oder 2 und
m ist die Zahl Null oder 1,
wobei die Summe von (m + n) gleich 2 ist und wobei die Verbindung der Formel (1) mindestens drei der im Molekül möglichen Reste entsprechend den oben definierten Formeln (2a) bis (2f) und der nachstehenden Formel (5) 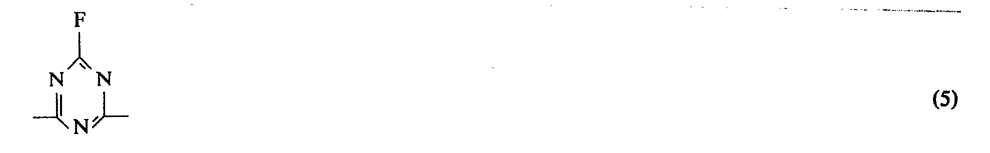 zwingend enthält;
M ist ein Wasserstoffatom oder das Äquivalent eines Metalls.

2. Verbindungen nach Anspruch 1 der allgemeinen Formel 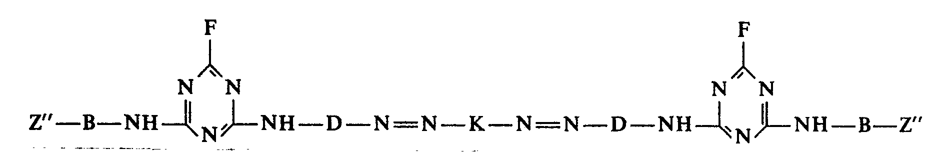 in welcher
B der Phenylenrest ist, der durch 1 Sulfogruppe oder durch 1 Methylgruppe oder Methoxygruppe oder 1 Chloratom oder 2 Methoxygruppen oder 1 Methoxygruppe und 1 Methylgruppe substituiert sein kann, wobei die beiden Formelreste B gleich oder verschieden voneinander sein können,
D der Phenylenrest ist, der durch 1 oder 2 Sulfogruppen oder 1 Methoxygruppe, 1 Methylgruppe oder 1 Chloratom oder durch 2 Methoxygruppen oder durch 1 Methoxygruppe und eine Methylgruppe substituiert sein kann, wobei die beiden Formelreste D gleich oder verschieden voneinander sein können,
K für den bivalenten Rest der als Kupplungskomponente dienenden doppel-ankuppelbaren 1-Amino-8-naphthol-3,6- oder -4,6-disulfonsäure steht und
Z" die β-Chloräthylsulfonyl, β-Acetoxyäthylsulfonyl-, β-Thiosulfatoäthylsulfonyl-, β-Phosphatoäthylsulfonyl-, β-Sulfatoäthylsulfonyl- oder die Vinylsulfonyl-Gruppe bedeutet.

3. Verbindungen nach Anspruch 1 der allgemeinen Formel 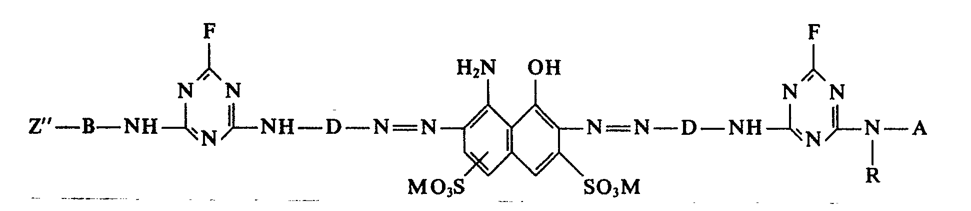 in welcher
A eine Methyl- oder Äthylgruppe, eine β-Hydroxyäthyl-, ,8-Sulfoäthyl-, Carboxyäthyl-, β-Sulfatoäthyl- oder Sulfophenylgruppe bedeutet,
R ein Wasserstoffatom, eine Methylgruppe oder eine Äthylgruppe ist und
M, B, D und Z" die in Anspruch 1 bzw. Anspruch 2 genannten Bedeutungen haben.

4. Verbindungen nach Anspruch 1 der allgemeinen Formel 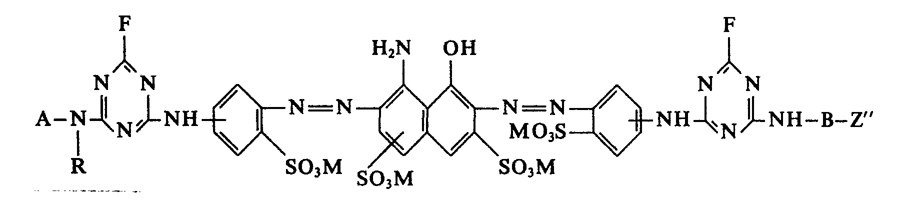 in welcher A, R, M, B, D und Z" die in Anspruch 3 genannten Bedeutungen haben.

5. Verbindunaen nach Ansoruch 1 der allaemeinen Formel 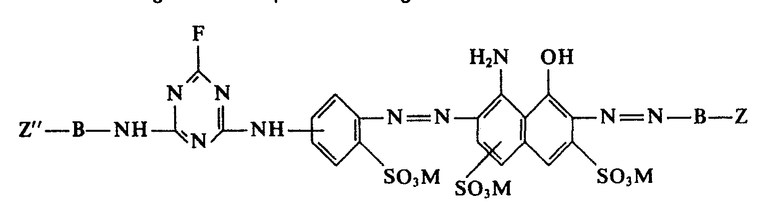 worin Z", B, Z und M die in Anspruch 1 bzw. 2 genannten Bedeutungen haben.

6. Verbindungen nach Anspruch 1 der allgemeinen Formel 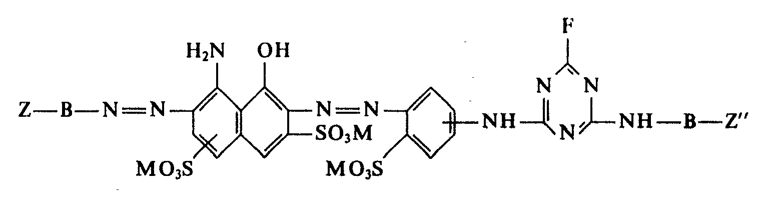 worin Z", B, Z und M die in Anspruch 1 bzw. 2 genannten Bedeutungen haben.

7. Verbindungen nach Anspruch 1 der allgemeinen Formel 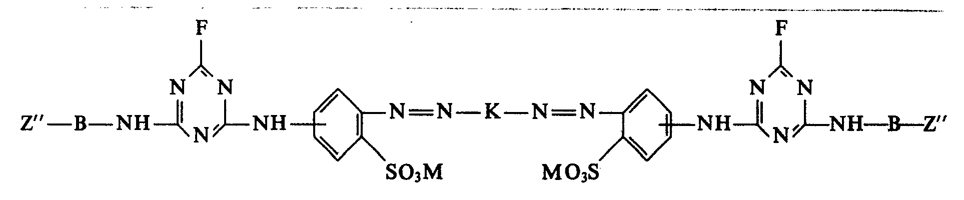 in welcher B, Z", K und M die in Anspruch 2 genannten Bedeutungen haben.

8. Verbindungen nach Anspruch 1 der allgemeinen Formel 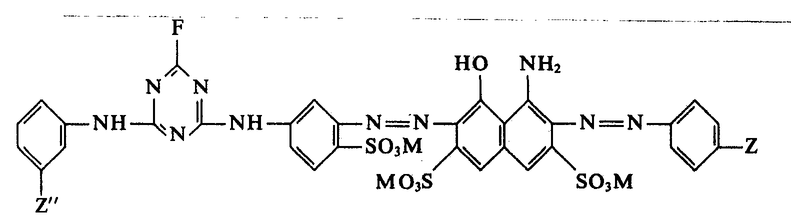 worin M, Z und Z" die in Anspruch 1 bzw. 2 genannten Bedeutungen haben.

9. Verbindungen nach Anspruch 1 der allgemeinen Formel 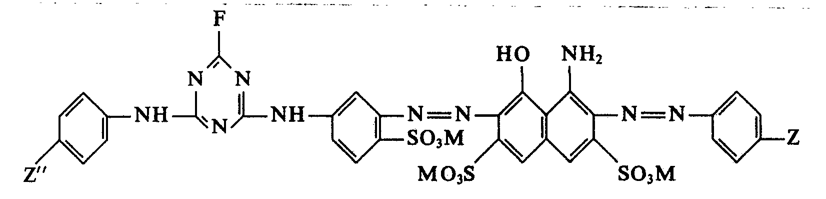 worin M, Z und Z" die in Anspruch 1 bzw. 2 genannten Bedeutungen haben.

10. Verbindungen nach einem der Ansprüche 1 bis 9, dadurch gekennzeichnet, daß Z und Z" die jl-Sulfatoäthylsulfonyl- oder die Vinylsulfonyl-Gruppe bedeuten.

11. Verbindungen nach einem der Ansprüche 1 bis 10, dadurch gekennzeichnet, daß M für Natrium oder Kalium steht.

12. Verfahren zur Herstellung der Disazoverbindungen der allgemeinen Formel (1) von Anspruch 1, dadurch gekennzeichnet, daß man in äquimolaren Mengen 1-Amino-8-naphthol-3,6-disulfonsäure oder 1-Amino-8-naphthol-4,6-disulfonsäure zunächst mit einer Diazoniumverbindung eines Amins der allgemeinen Formel (6) 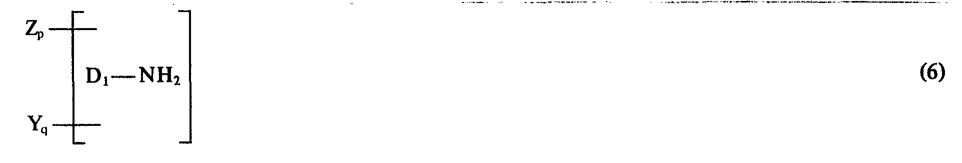 in welcher D₁, Z und Y die in Anspruch 1 genannten Bedeutungen haben und p und q jedes für die Zahl Null oder 1 steht, in stark saurem bis schwach saurem Bereich und nachfolgend mit einer Diazoniumverbindung eines Amins der allgemeinen Formel (7) 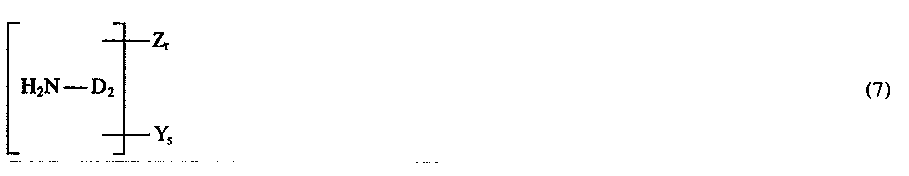 in welcher D_{z}, Z und Y die in Anspruch 1 genannten Bedeutungen besitzen und r und s jedes für die Zahl Null oder 1 steht, in schwach saurem bis schwach alkalischem Bereich kuppelt, wobei man die Amine der allgemeinen Formeln (6) und (7) so auswählt, daß die Summe von (p+q) gleich 1, die Summe von (r+s) gleich 1, die Summe von (p+r) gleich Null oder 1 und die Summe von (q +s) gleich 1 oder 2 ist und die Diazokomponenten der allgemeinen Formeln (6) und (7) gleich oder voneinander verschieden sein können.

13. Verwendung der Disazoverbindungen von Anspruch 1 als Farbstoffe.

14. Verwendung nach Anspruch 11 zum Färben von hydroxy-, amino- und/oder carbonamidgruppenhaltigem Material, insbesondere Fasermaterial.

15. Verfahren zum Färben von hydroxy-, amino- und/oder carbonamidgruppenhaltigem Material, insbesondere Fasermaterial, bei welchem man einen Farbstoff auf das Material aufbringt oder in das Material einbringt und ihn darauf oder gegebenenfalls darin, gegebenenfalls mittels Wärme und/oder mit Hilfe eines säurebindenden Mittels fixiert, dadurch gekennzeichnet, daß man als Farbstoff eine Verbindung der in Anspruch 1 genannten und definierten allgemeinen Formel (1) einsetzt.

## Claims

1. Water-soluble disazo compounds of the general formula (1) 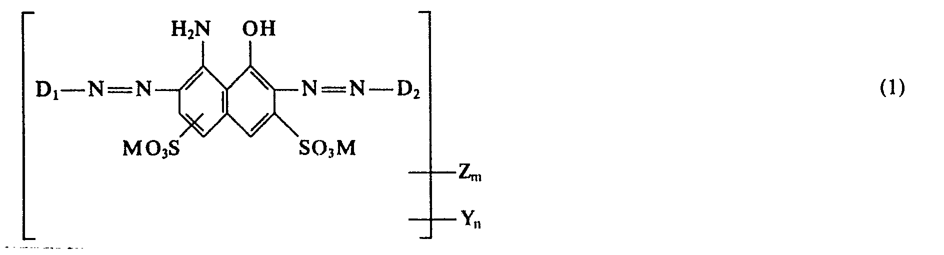 in which:
Dᵢ and D₂ each represent the phenyl radical or a naphthyl radical, both of which are substituted by a group of the formula moiety Y or formula moiety Z, defined below, and can additionally be substituted by one or two substituents from the group comprising sulfo, chlorine, bromine, lower alkyl and lower alkoxy, and Dₗ and D₂ can have meanings which are identical to or different from one another;
Z is a group of the formula (2a), (2b), (2c), (2d), (2e) or (2f) 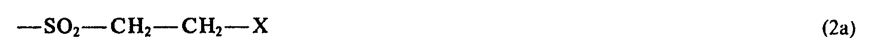 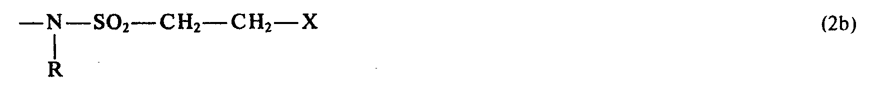 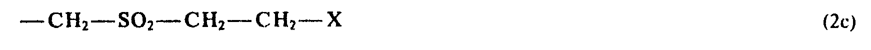 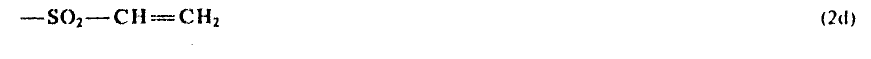 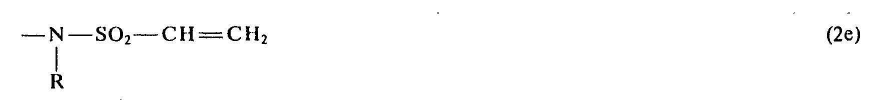 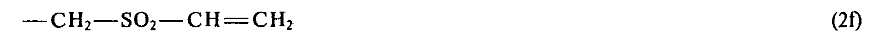 in which
R denotes an alkyl group having 1 to4 C-atoms and
X denotes a chlorine atom, the acetoxy group, the thiosulfato group, the phosphato group or the sulfato group;
Y is a radical of the formula (3) 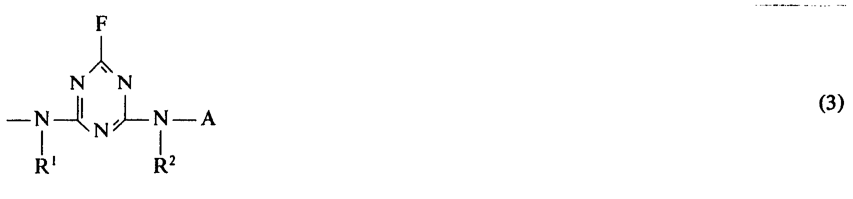 in which
R^{l} is a hydrogen atom or an alkyl group having 1 to 4 C-atoms and
R² is a hydrogen atom or an alkyl group having 1 to 4 C-atoms,
and R^{l} and R² can have meanings which are identical to or different from one another, and
A is a hydrogen atom or an alkyl group having 1 to 6 C-atoms which can be substituted, or is the phenyl radical which can be substituted by substituents from the group comprising methyl, ethyl, methoxy, ethoxy, chlorine, bromine, carboxy, sulfo, carbamoyl and sulfamoyl, or is a radical of the formula (4) 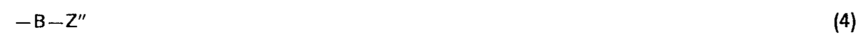 in which
B is the penylene radical or a naphthylene radical, both of which can be substituted by one or two substitutents chosen from the set comprising one sulfo group, one chlorine atom, one or two methyl or ethyl groups and one or two methoxy or ethoxy groups, and
Z" has one of the meanings mentioned for Z;
n is the number 1 or 2 and
m is the number zero or 1,
and the sum (m + n) is equal to 2, and the compound of the formula (1) contains mandatorily at least three of the radicals which can be present in the molecule and correspond to the above-defined formulae (2a) to (2f) and to the following formula (5) 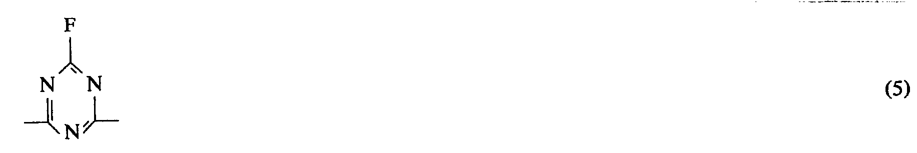 M is a hydrogen atom or the equivalent of a metal.

2. Compounds according to claim 1, of the general formula 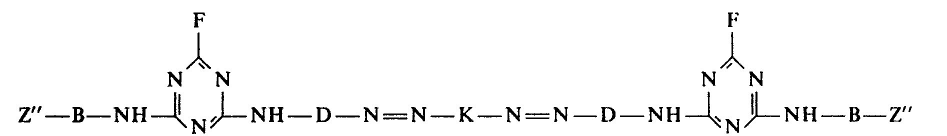 in which
B is the phenylene radical which can be substituted by 1 sulfo group or by 1 methyl group or methoxy group or 1 chlorine atom or 2 methoxy groups or 1 methoxy group and 1 methyl group, and the two formula moieties B can be identical to or different from one another,
D is the phenylene radical which can be substituted by 1 or 2 sulfo groups or 1 methoxy group, 1 methyl group or 1 chlorine atom or by 2 methoxy groups or by 1 methoxy group and one methyl group, and the two formula moieties D can be identical to or different from one another,
K represents the bivalent radical or 1-amino-8-naphthol-3,6- or -4,6-disulfonic acid which serve as the coupling component coupling twice, and
Z" denotes the β-chloroethylsulfonyl β-acetoxyethylsulfonyl, β-thiosulfatoethylsulfonyl, β-phospha- toethylsulfonyl, β-sulfatoethylsulfonyl or the vinylsulfonyl group.

3. Compounds according to claim 1, of the general formula 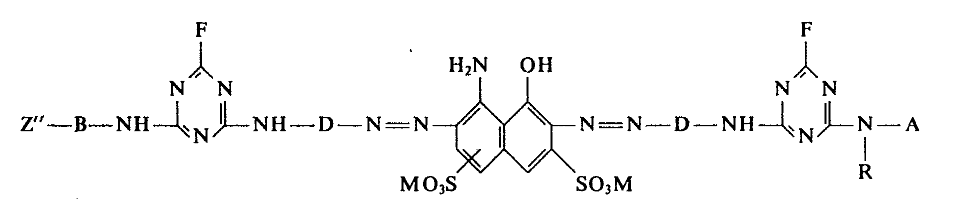 in which
A denotes a methyl or ehtyl group, a β-hydroxyethyl, γ-sulfoethyl, carboxyethyl, β-sulfatoethyl or sulfophenyl group,
R is a hydrogen atom or a methyl group or an ethyl group and
M, B, D and Z" have the meanings mentioned in claim 1 or claim 2, respectively.

4. Compounds according to claim 1, of the general formula 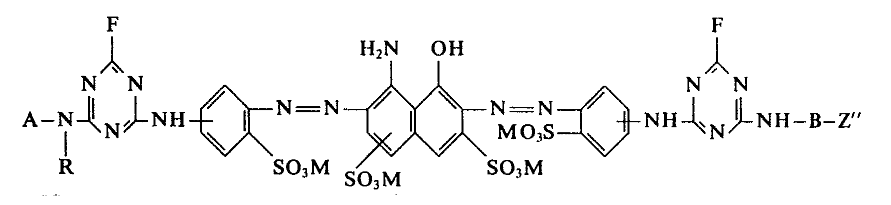 in which A, R, M, B, D and Z" have the meanings mentioned in claim 3.

5. Compounds according to claim 1, of the general formula 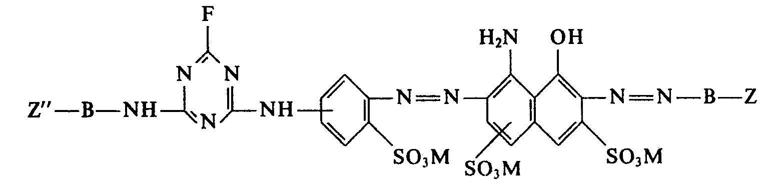 in which Z", B, Z and M have the meanings mentioned in claim 1 or 2, respectively.

6. Compounds according to claim 1, of the general formula 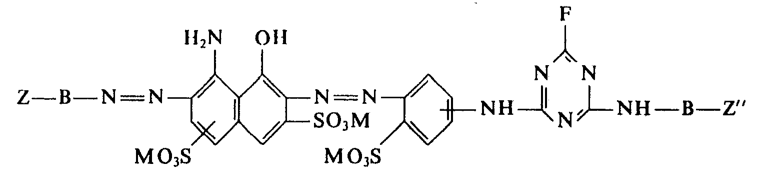 in which Z", B, Z and M have the meanings mentioned in claim 1 or 2, respectively.

7. Compounds according to claim 1, of the general formula 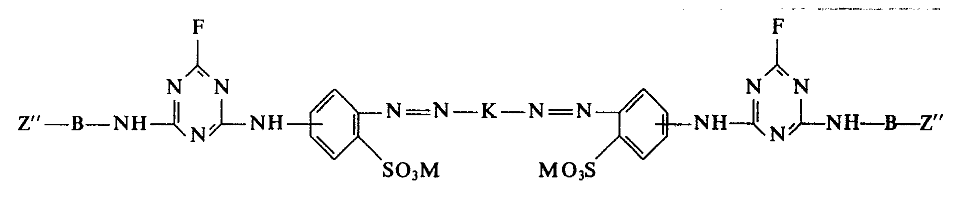 in which B, Z", K and M have the meanings mentioned in claim 2.

8. Compounds according to claim 1, of the general formula 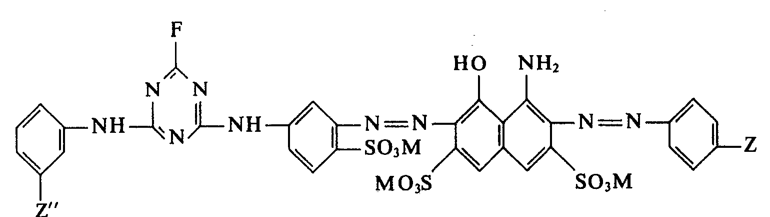 in which M, Z and Z" have the meanings mentioned in claim 1 or 2, respectively.

9. Compounds according to claim 1, of the general formula 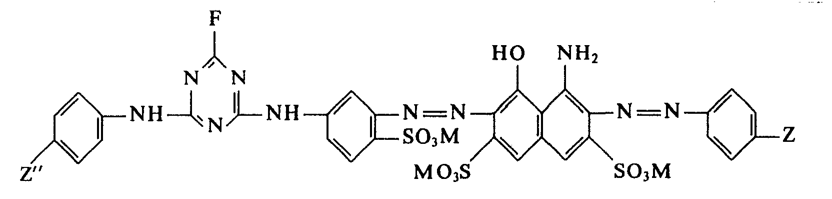 in which M, Z and Z" have the meanings mentioned in claim 1 or 2, respectively.

10. Compounds according to any of claims 1 to 9, characterized by that Z and Z" denote the β-sulfatoethylsulfonyl or the vinylsulfonyl group.

11. Compounds according to any of claims 1 to 10, characterized by that M represents sodium or potassium.

12. A process for preparing the disazo compounds of the formula (1) of claim 1, characterized by that I-amino-8-naphthol-3,6-disulfonic acid or 1-amino-8-naphthol-4,6-disulfonic acid is first coupled, in equimolar amounts, with a diazonium compound of an amine of the general formula (6) 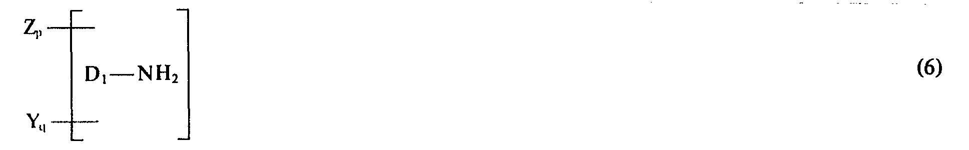 in which D₁, Z and Y have the meanings mentioned in claim 1 and p and q each represent the numbers zero or 1, in strongly acid to weakly acid range and subsequently with a diazonium compound of an amine of the general formula (7) 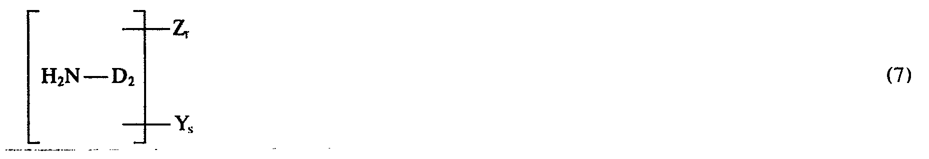 in which D₂, Z and Y have the meaning mentioned in claim 1 and r and s each represent the number zero or 1, in weakly acid to weakly alkaline range,
the amines of the general formulae (6) and (7) being so chosen that the sum (p+q) is equal to 1, the sum (r+s) is equal to 1, the sum (p+r) is equal to zero or 1 and the sum (q + s) is equal to 1 or 2, and the diazo components of the formulae (6) and (7) can be identical to or different from one another.

13. The use of the disazo compounds of claim 1 as dyestuffs.

14. The use according to claim 11, for coloring material, in particular fiber material, containing hydroxy, amino and/or carbonamide groups.

15. A process for coloring a material, in particular fiber material, containing hydroxy, amino and/or carbonamide groups, in which process a dyestuff is applied to the material or introduced into the material and then fixed thereon or, if appropriate, therein, optionally by means of heat and/or with the aid of an acid-binding agent, characterized by that a compound of the general formula (1) mentioned and defined in claim 1, is used as the dyestuff.

## Revendications

1. Composés disazoïques solubles dans l'eau qui répondent à la formule générale 1: 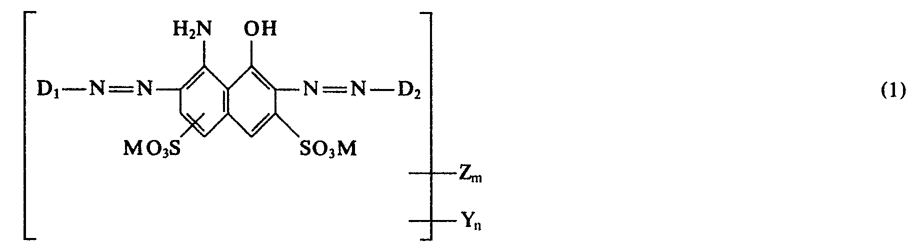 dans laquelle
Dₗ et D₂ représente chacun un radical phényle ou un radical naphthyle, chacun de ces radicaux portant un radical Y ou Z tel que défini ci-dessous et pouvant en outre porter un ou deux substituants pris dans l'ensemble constitué par le sulfo, le chlore, le brome, les alkyles inférieurs et les alcoxy inférieurs,
Dₗ et D₂ pouvant être identiques l'un à l'autre ou différents l'un de l'autre,
Z représente un radical répondant à l'une des formules 2a, 2b, 2c, 2d, 2e et 2f: 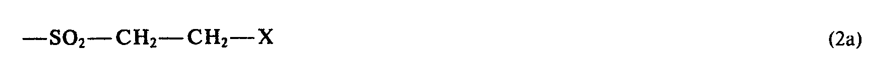 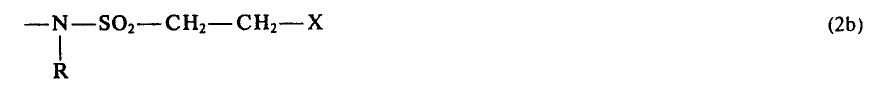 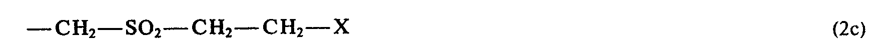 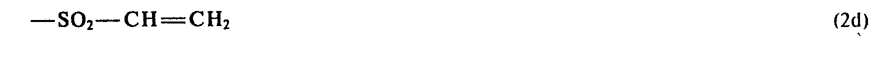 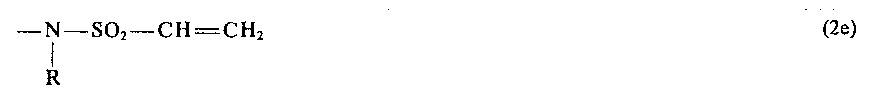 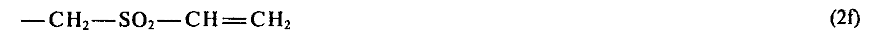 dans lesquelles
R représente un radical alkyle contenant de 1 à 4 atomes de carbone et
X représente un atome de chlore ou un radical acétyloxy, thiosulfato, phosphato ou sulfato,
Y représente un radical répondant à la formule 3: 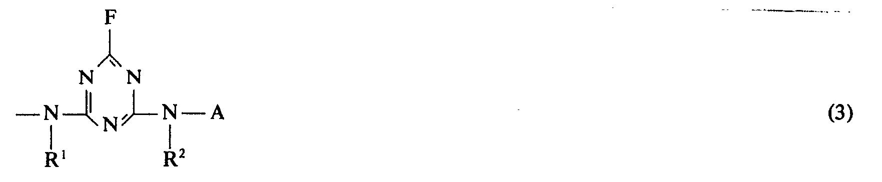 dans laquelle
R¹ représente un atome d'hydrogène ou un alkyle en C₁―C₄,
R² représente un atome d'hydrogène ou un alkyle en C₁―C₄,
R¹ et R² pouvant être identiques l'un à l'autre ou différents l'un de l'autre, et
A représente un atome d'hydrogène, un alkyle en C₁―C₆ éventuellement substitué, un phényle éventuellement porteur de substituants pris dans l'ensemble constitué par les radicaux méthyle, éthyle, méthoxy, éthoxy, chloro, bromo, carboxy, sulfo, carbamoyle et sulfamoyle, ou représente un radical répondant à la formule 4: 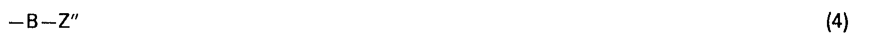 dans laquelle
B représente un radical phényléne ou un radical naphtylène, chacun d'eux pouvant porter un ou deux substituants choisis dans l'ensemble constitué par un radical sulfo, un atome de chlore, un ou deux radicaux méthyle ou éthyle et un ou deux radicaux méthoxy ou éthoxy, et
Z" a l'une des significations qui ont été données pour Z,
n représente le nombre 1 ou le nombre 2,
m représente le nombre 0 ou le nombre 1,
la somme (m + n) étant égale à 2 et le composé de formule 1 contenant obligatoirement au moins trois des radicaux dont la présence est possible dans la molécule et qui répondent aux formules 2a à 2f définies ci-dessus et à la formule 5 suivante: 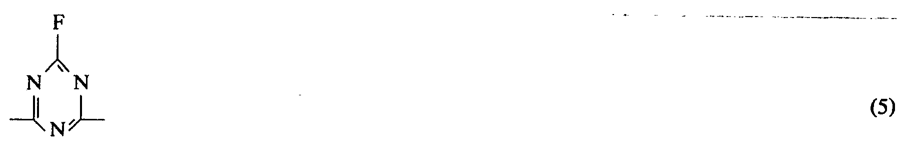 et
M représente un atome d'hydrogène ou l'équivalent d'un métal.

2. Composés selon la revendication 1 qui répondent à la formule générale suivante: 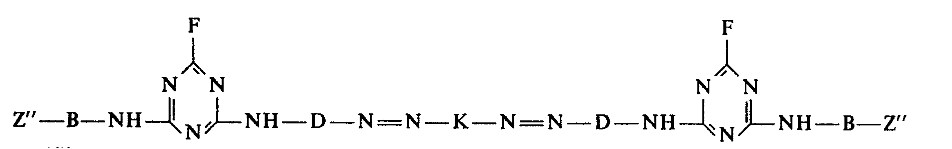 dans laquelle
B représente un radical phénylène éventuellement porteur d'un radical sulfo ou d'un radical méthyle ou méthoxy ou d'un atome de chlore ou de deux radicaux méthoxy ou d'un radical méthoxy et d'un radical méthyle, les deux symboles B pouvant avoir la même signification ou des significations différentes,
D représente un radical phénylène éventuellement porteur d'un ou deux radicaux sulfo ou d'un radical méthoxy, d'un radical méthyle ou d'un atome de chlore ou de deux radicaux méthoxy ou d'un radical méthoxy et d'un radical méthyle, les deux symboles D pouvant avoir la même signification ou des significations différentes,
K représente le radical bivalent de l'acide amino-1 hydroxy-8 naphtalène-disulfonique-3,6 ou disulfonique-4,6 capable de copuler deux fois et qui sert de copulant, et
Z" représente un radical chloro-2 éthylsulfonyle, acétoxy-2 éthylsulfonyle, thiosulfato-2 éthylsulfonyle, phosphato-2 éthylsulfonyle, sulfato-2 éthylsulfonyle ou vinylsulfonyle.

3. Composés selon la revendication 1 qui répondent à la formule générale suivante: 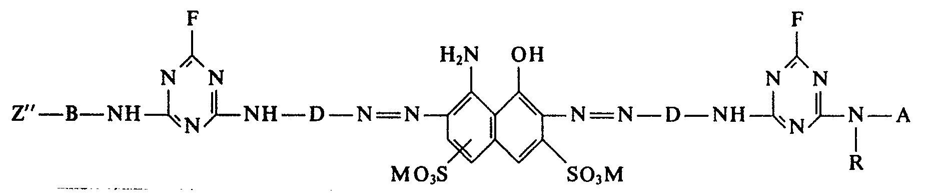 dans laquelle
A représente un radical méthyle, éthyle, hydroxy-2 éthyle, sulfo-2 éthyle, carboxyéthyle, sulfato-2 éthyle ou sulfophényle,
R représente un atome d'hydrogène ou un radical méthyle ou éthyle et
M, B, D et Z" ont les significations qui ont été indiquées à la revendication 1 ou à la revendication 2.

4. Composés selon la revendication 1 qui répondent à la formule générale suivante: 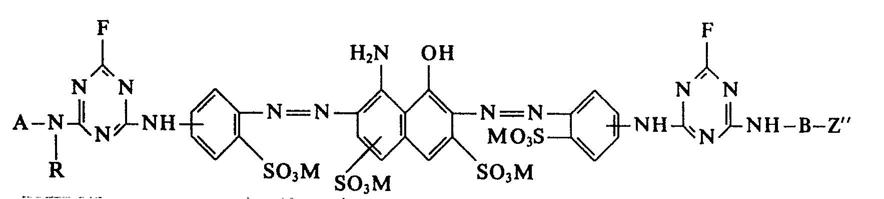 dans laquelle A, R, M, B, D et Z" ont les significations données à la revendication 3.

5. Composés selon la revendication 1 qui répondent à la formule générale suivante: 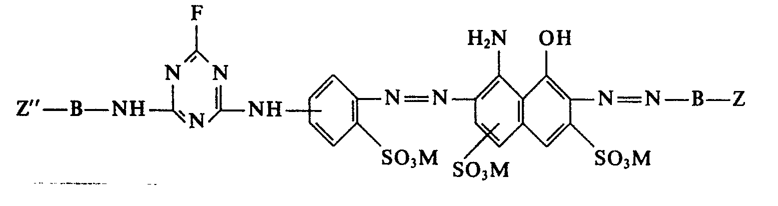 dans laquelle Z" , B, Z et M ont les significations données à la revendication 1 ou à la revendication 2.

6. Composés selon la revendication 1 qui répondent à la formule générale suivante: 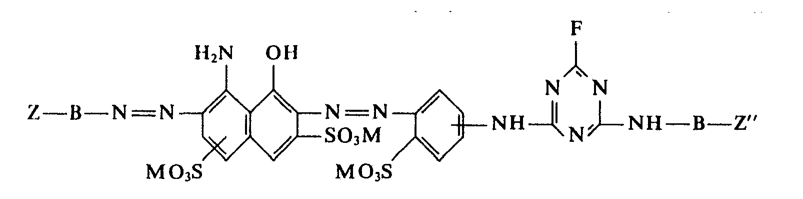 dans laquelle Z", B, Z et M ont les significations données à la revendication 1 ou à la revendication 2.

7. Composés selon la revendication 1 qui répondent à la formule générale suivante: 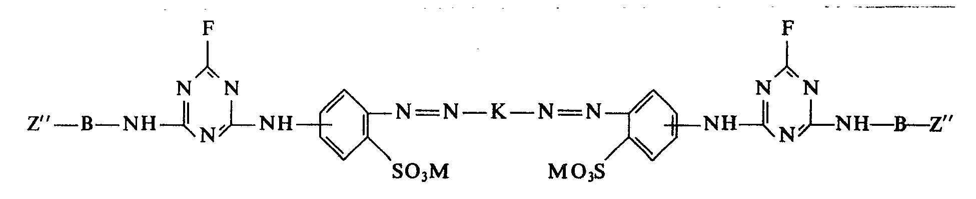 dans laquelle B, Z", K et M ont les significations données à la revendication 2.

8. Composés selon la revendication 1 qui répondent à la formule générale suivante: 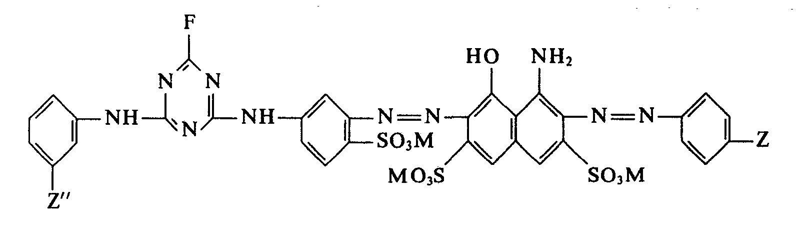 dans laquelle M, Z et Z" ont les significations données la revendication 1 ou la revendication 2.

9. Composés selon la revendication 1 qui répondent à la formule générale suivante: 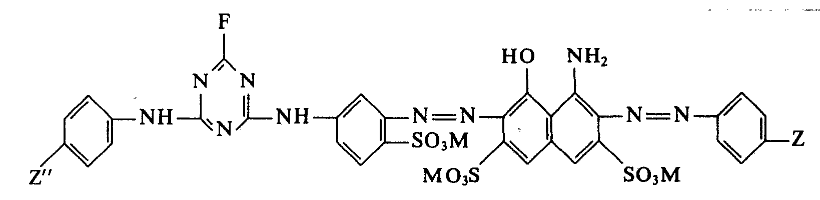 dans laquelle M, Z et Z" ont les significations données à la revendication 1 ou à la revendication 2.

10. Composés selon l'une quelconque des revendications 1 à 9, caractérisés en ce que Z et Z" représentent un radical sulfato-2 éthylsulfonyle ou un radical vinylsulfonyle.

11. Composés selon l'un quelconque des revendications 1 à 10, caractérisés en ce que M représente le sodium ou le potassium.

12. Procédé de préparation de composés disazoïques de formule générale 1 selon la revendication 1, procédé caractérisé en ce qu'on copule, en des quantités équimolaires, l'acide amino-1 hydroxy-8 naphtalène-disulfonique-3,6 ou l'acide amino-1 hydroxy-8 naphtalène-disulfonique-4,6 d'abord avec un composé de diazonium d'un amine répondant à la formule générale 6: 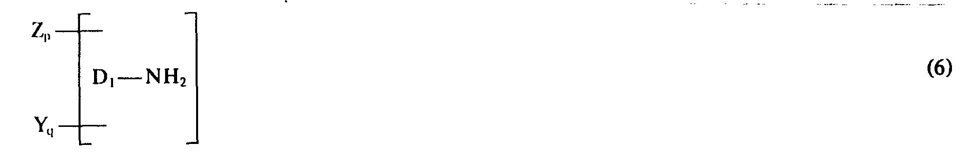 dans laquelle 0₁, Z et Y ont les significations données à la revendication 1 tandis que p et q représentent chacun le nombre 0 ou le nombre 1, en milieu très acide à légèrement acide, puis avec un composé de diazonium d'une amine répondant à la formule générale 7: 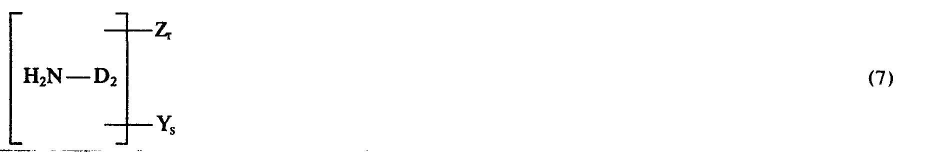 dans laquelle D₂, Z et Y ont les significations données à la revendication 1 tandis que r et s représentent chacun le nombre 0 ou le nombre 1, en milieu légèrement acide à légèrement alcalin,
les amines de formules générales 6 et 7 étant choisies de telle façon que la somme (p + q) soit égale à 1, la somme (r+ s) soit égale à 1, la somme (p + r) soit égale à 0 ou à 1 et la somme (q + s) soit égale à 1 ou 2, et les composantes de diazotation de formules générales 6 et 7 pouvant être identiques l'une à l'autre ou différentes l'une de l'autre.

13. Application des composés disazoïques selon la revendication 1 comme colorants.

14. Application selon la revendication 11 caractérisé en ce qu'on teint des matières contenant des radicaux hydroxy, amino et/ou carbamoyles, plus spécialement des matières fibreuses de ce type.

15. Procédé pour teindre des matières contenant des radicaux hydroxy, amino et/ou carbamoyles, plus particulièrement des matières fibreuses de ce type, selon lequel on applique un colorant sur la matière ou on l'introduit dans la matière, après quoi on le fixe sur celle-ci ou éventuellement dans celle-ci, le cas échéant au moyen de la chaleur et/ou d'un accepteur d'acides, ce procédé étant caractérisé en ce qu'on utilise, comme colorant, un composé répondant à la formule générale 1 qui a été représentée et définie à la revendication 1.
